# EUROPEAN PATENT SPECIFICATION

(11) **EP 2 548 518 B1**
(45) Date of publication and mention of the grant of the patent: **06.09.2017**
(21) Application number: 12175097.0
(22) Date of filing: 05.07.2012
(51) Int. Cl.: A61B 17/00

(54) **Vascular hole closure delivery device**
Vorrichtung zur Bereitstellung eines Gefäßlochverschlusses
Dispositif de fourniture de fermeture d'orifice vasculaire

(30) Priority: 02.04.2012 US 201213437146; 20.07.2011 US 201161509829 P
(43) Date of publication of application: 23.01.2013
(73) Proprietor: Rex Medical, L.P., Conshohocken, PA 19428 (US)
(72) Inventor: DeFonzo, Stephan A., Wayne, PA 19087 (US); Tarmin, James S, Philadelphia, PA 19123 (US); Anidharan, Thanu, Downington, PA 19335 (US)
(74) Representative: Jackson, Derek Charles

(56) References cited:
- EP-A1- 2 412 317
- WO-A1-95/32670
- WO-A1-2004/098418
- WO-A1-2006/093970
- US-A- 5 433 727
- US-A- 5 507 754
- US-A1- 2009 210 004
- US-A1- 2009 216 267
- US-A1- 2012 078 294
- US-B1- 6 231 561

## Description

### Technical Field

This application relates to a delivery device for a vascular device and more particularly to a delivery device for a vascular hole closure device.

### Background Art

During certain types of vascular surgery, catheters are inserted through an incision in the skin and underlying tissue to access the femoral artery in the patient's leg. The catheter is then inserted through the access opening made in the wall of the femoral artery and guided through the artery to the desired site to perform surgical procedures such as angioplasty or plaque removal. After the surgical procedure is completed and the catheter is removed from the patient, the access hole must be closed. This is difficult not only because of the high blood flow from the artery, but also because there are many layers of tissue that must be penetrated to reach the femoral artery.

Several approaches to date have been used to close femoral access holes. In one approach, manual compression by hand over the puncture site is augmented by a sandbag or weight until the blood coagulates. With this approach, it can take up to six hours for the vessel hole to close and for the patient to be able to ambulate. This inefficiency increases the surgical procedure time as well as the overall cost of the procedure since the hospital staff must physically maintain pressure and the patient's discharge is delayed because of the inability to ambulate.

In another approach to close the vessel puncture site, a clamp is attached to the operating table and the patient's leg. The clamp applies pressure to the vessel opening. The patient, however, must still be monitored to ensure the blood is coagulating, requiring additional time of the hospital staff and increasing the cost of the procedure.

To avoid the foregoing disadvantages of manual pressure approaches, suturing devices have been developed. One such suturing device, sold by Abbott, advances needles adjacent the vessel wall opening and pulls suture material outwardly through the wall adjacent the opening. The surgeon then ties a knot in the suture, closing the opening. One difficulty with the procedure involves the number of steps required by the surgeon to deploy the needles, capture the suture, withdraw the suture, and tie the knot and secure the suture. Moreover, the surgeon cannot easily visualize the suture because of the depth of the femoral artery (relative to the skin) and essentially ties the suture knot blindly or blindly slips a pre-tied knot into position. Additionally, the ability to tie the knot varies among surgeons; therefore success and accuracy of the hole closure can be dependent on the skill of the surgeon. Yet another disadvantage of this suturing instrument is that the vessel opening is widened for insertion of the instrument, thus creating a bigger opening to close in the case of failure to deliver the closure system. It is also difficult to pass the needle through calcified vessels.

U.S. Patent No. 4,744,364 discloses another approach for sealing a vessel puncture in the form of a device having an expandable closure member with a filament for pulling it against the vessel wall. The closure member is held in place by a strip of tape placed on the skin to hold the filament in place. However, the closure device is still subject to movement which can cause leakage through the puncture. Additionally, if the suture becomes loose, the closure member is not retained and can flow downstream in the vessel. Moreover, since the suture extends through the skin, a potential pathway for infection is created. The closure device in U.S. Patent No. 5,545,178 includes a resorbable collagen foam plug located within the puncture tract. However, since coagulation typically takes up to twenty minutes and blood can leak in between the plug and tissue tract, manual pressure must be applied to the puncture for a period of time, until the collagen plug expands within the tract.

It would therefore be advantageous to provide a device which would more quickly and effectively close openings (punctures) in vessel walls. Such device would advantageously avoid the aforementioned time and expense of applying manual pressure to the opening, simplify the steps required to close the opening, avoid widening of the opening, and more effectively retain the closure device in the vessel.

Commonly assigned U.S. Patent No. 7,662,161 discloses effective vascular hole closure devices which have the foregoing advantages. It would be further advantageous to provide a vascular hole closure device which is adjustable to accommodate different tissue thicknesses and applies a more constant clamping/retaining force between the intravascular and extravascular components of the device irrespective of tissue thickness. Such adjustability is achieved in copending commonly assigned application serial no. 12/854,988, filed August 12, 2010, (hereinafter the '988 application).

The need exists for an effective delivery instrument to deliver the closure device of the '988 application to the target site to close the vascular access hole.

US5507754 describes an apparatus for elevating, approximating and/or restraining internal organs or structures and includes an anchoring device having an elongated suture with an anchor at each end thereof and a cinching member therebetween for adjusting the length of the suture, an apparatus for applying the anchoring device, and an apparatus for drawing the suture relative to the cinching member after it has been emplaced.

WO2006/093970 describes a structure that can be used to provide an anchor in or adjacent to a patient's soft body tissue (rather than bone) and which includes an annular array of flexible members that extend between two axially spaced but aligned tubular members. The space between the tubular members can be selectively changed to affect the amount by which portions of the flexible members between the tubular members project radially outward relative to the tubular members. When the flexible members project radially out, the structure resists axial movement relative to adjacent tissue and therefore acts as an anchor. When the flexible members do not project radially out, they permit axial movement of the structure through adjacent tissue.

US2009/0216267 describes closure devices with a rapidly dissolving anchor and systems for delivering such closure devices. An example closure device for closing an opening in a body lumen may include a plug, a rapidly dissolving anchor, and a suture coupling the plug to the anchor. The rapidly dissolving anchor may be configured to dissolve within the body lumen within about 30 days or less. At least a portion of the plug may be disposed adjacent an exterior surface of the body lumen. At least a portion of the rapidly dissolving anchor may be disposed within the body lumen.

US2009/0210004 describes a device for closing an aperture in a vessel wall comprising a covering member having a longitudinal axis and positionable inside the vessel against the internal opening of the aperture and a first spherical retainer positionable external of the vessel. The covering member has a dimension to prevent egress of fluid through the aperture.

EP2412317 describes a suturing device which includes an elongate shaft defining a longitudinal axis, a trajectory guide translatably mounted on the elongate shaft and an introducer guard member operatively coupled to a distal portion of the elongate shaft. The introducer guard member includes at least one arm member which is movable between a first position in which the arm member is retracted, substantially in alignment with the longitudinal axis and a second position in which the at least one arm member is deployed. The introducer guard member includes an attaching member for releasably retaining a suture. The trajectory guide defines at least one bore therethrough. The bore obliquely extends through the trajectory guide and defines an angle with respect to the longitudinal axis. The bore aligns with the attaching member when the introducer guard member is in the second position. Document US6231561 discloses a surgical instrument for delivering into a vessel a vascular hole closure device according to the preamble of claim 1.

### Summary of invention

The invention is as disclosed in the appended set of claims. According to the present invention there is provided a surgical delivery instrument for delivering into a vessel a vascular hole closure device having a hole covering member, the delivery instrument comprising a housing, an advancer movable within the housing, and a plunger, the advancer having a first portion and a distal portion hingedly connected to the first portion and forming a casing for supporting a hole covering member, wherein the housing includes an angled inner surface engageable by the casing for pivoting the same within the housing, distal movement of the advancer pivoting the casing within the housing from an angled position relative to the first portion to a more aligned position relative to the first portion, and wherein the plunger is movable subsequent to the movement of the advancer to advance a hole covering member from the housing.

The plunger may include a tube and a handle portion positioned proximally of the tube, and movement of the handle portion may distally advance the tube within a lumen of the advancer.

The surgical delivery instrument may further comprise a distal stop to limit distal movement of the advancer.

The surgical delivery instrument may further comprise a first actuator for moving the advancer distally, the first actuator movable independently of the plunger.

The plunger may be configured such that initial advancement thereof may move the advancer distally and subsequent advancement thereof may advance the covering member from the housing.

The surgical delivery instrument may further comprise first and second rails operatively connecting the advancer and the plunger, wherein the first and second rails are operatively disassociated from the plunger when the advancer is moved to a distal position into contact with a stop.

The present invention also relates to a kit comprising a surgical delivery instrument as hereinbefore defined and a vascular hole closure device including a hole covering member, wherein the hole closure device further includes first and second flexible members, the first flexible member having a first engagement member and the second flexible member having a second engagement member, wherein the plunger has first and second longitudinally extending openings and first and second engaging portions, the first engaging portion limiting movement of the first engagement member and the second engaging portion limiting movement of the second engagement member, wherein the first engagement member is held by the first engaging portion until a predetermined force is applied to the first engagement member during placement of the closure device at a target site and the second engagement member is held by the second engaging portion until a predetermined force is applied to the second engagement member during placement of the closure device at the target site.

The first engaging portion may comprise a first opening in a first grommet aligned with the first longitudinally extending opening and the second engaging portion may comprise a second opening in a second grommet aligned with the second longitudinally extending opening. The first grommet may have a third opening and the second grommet may have a fourth opening, wherein the first engagement member is passable through the first opening when a first force is applied and subsequently passable through the third opening when a subsequent force is applied and the second engagement member is passable through the second opening when a second force is applied and subsequently passable through the fourth opening when a subsequent force is applied.

The first flexible member may be a suture and the second flexible member may be a suture, and the delivery instrument may further comprise a cutting member positioned within the housing for automatically severing the sutures.

The covering member of the vascular hole closure device may be at a distal end for positioning internal of a vessel and the device may further include a first and a second retainer for positioning external of the vessel, the first flexible member extending between the covering member and the first retainer and the first engagement member positioned at a proximal portion of the first flexible member, and the second flexible member extending between the covering member and the second retainer and the second engagement member positioned at a proximal portion of the second flexible member, wherein proximal movement of the delivery instrument advances the first retainer and the second retainer toward the covering member.

### Brief description of drawings

Preferred embodiment(s) of the present disclosure are described herein with reference to the drawings wherein:
Figure 1 is a perspective view of a first embodiment of the hole closure delivery instrument of the present disclosure;
Figure 2 is an exploded view of the advancer portion of the delivery instrument of Figure 1;
Figure 2A is an exploded view of the handle (plunger) portion of the delivery instrument of Figure 1;
Figure 2B is an enlarged view of the area of detail of Figure 2;
Figure 3 is a longitudinal cross-sectional view taken along line 3-3 of Figure 1 showing the advancer in the initial position;
Figure 3A is a longitudinal cross-sectional view taken along line 3A-3A of Figure 1 showing the handle portion in the initial position;
Figure 3B is an enlarged view of the area of detail of Figure 3;
Figure 4 is a transverse cross-sectional view taken along line 4-4 of Figure 3;
Figure 5 is a transverse a cross-sectional view taken along line 5-5 of Figure 3;
Figure 6 is a transverse cross-sectional view taken along line 6-6 of Figure 3A;
Figure 7 is a perspective view of the advancer portion illustrating initial movement of the sliding tab;
Figure 8 is a view similar to Figure 3B showing initial movement of the sliding tab;
Figure 8A is a longitudinal cross-sectional view similar to Figure 3 showing initial movement of the sliding tab to advance the advancer tube and corresponding to the position of Figure 7, and further showing the sheath connected to the instrument;
Figure 8B illustrates the sliding tab adjacent the lockout;
Figure 8C illustrates the sliding tab advanced past the lockout;
Figure 9 is a perspective view similar to Figure 7 showing the sliding tab in the distal position to fully advance the advancer tube;
Figure 10 is a longitudinal cross-sectional view similar to Figure 8A showing the sliding tab in the distal position to fully advance the advancer tube and corresponding to the position of Figure 9;
Figure 11 is a perspective view similar to Figure 9 illustrating the sliding tab in the distal position and the handle portion advanced to the distal position to deploy the hole closure device in the vessel;
Figure 12 is an enlarged view of the area of detail of Figure 11;
Figure 13 is a longitudinal cross-sectional view similar to Figure 10 showing the sliding tab in the distal position and the handle portion advanced to the distal position, corresponding to the position of Figure 11;
Figure 14 is a perspective view illustrating initial retraction of the delivery instrument to further deploy the hole closure;
Figure 15 is an exploded view of a portion of the handle portion (plunger) showing a first housing half section with initial movement of the second engagement member and the first engagement member engaged with the lower opening of the distal blocking element in the central housing;
Figure 16 is a longitudinal cross-sectional view of the handle portion of the delivery instrument corresponding to the position of Figure 15 illustrating proximal movement of the handle portion of the delivery instrument to advance the first retainer of the closure device toward the covering member;
Figure 17 is an exploded view of a portion of the handle portion showing the first housing half section with further movement of the first engagement member in the lower channel of the central housing and the second engagement member engaged with the proximal blocking element in the lower channel of the first housing;
Figure 18 is a longitudinal cross-sectional view of the handle portion of the delivery instrument corresponding to the position of Figure 17 illustrating further proximal movement of the handle portion of the delivery instrument to advance the second retainer of the closure device toward the covering member;
Figure 19 is a view similar to Figure 15 illustrating the first engagement member in the curved channel of the central housing and the second engagement member engaged with the proximal blocking member at the upper opening;
Figure 20 is a cross-sectional view of the proximal end of the handle portion of the delivery instrument corresponding to the position of Figure 19 and showing further advancement of the second retainer of the closure device toward the covering member;
Figure 21 is a view similar to Figure 19 illustrating the first engagement member engaging an upper opening in the distal blocking member and the second engagement member in the upper channel of the central housing;
Figure 22 is a cross-sectional view of the proximal end of the handle portion of the delivery instrument corresponding to the position of Figure 21 and showing further proximal movement of the handle portion of the delivery instrument to further advance the first retainer of the closure device toward the covering member;
Figure 23 is a perspective view of a region of the handle portion showing the sutures adjacent the cutting blade;
Figure 24 is a cross-sectional view corresponding to the position of the second suture in Figure 23;
Figure 25 is a perspective view similar to Figure 23 showing the sutures engaged with the cutting blade;
Figure 26 is a cross-sectional view similar to Figure 24 corresponding to the position of the second suture in Figure 25;
Figure 27 is a perspective view of a hole closure delivery instrument in accordance with an alternate embodiment of the present disclosure;
Figure 28 is a cross-sectional view taken along line 28-28 of Figure 27;
Figure 29 is a front view of the delivery instrument of Figure 27;
Figure 30A is an exploded view of the advancer portion of the delivery instrument of Figure 27;
Figure 30B is an exploded view of the handle portion (plunger) of the delivery instrument of Figure 27;
Figures 31A, 31B and 31C are longitudinal cross-sectional views taken along lines 31A-31A, 31B-31B and 31C-31C of Figure 27 showing the delivery instrument (handle portion and advancer) in the initial position;
Figure 32 is a perspective view of a section of the delivery instrument of Figure 27 showing removal of the locking tab;
Figure 33 is a partially exploded view of the advancer portion with the advancer in the initial position (only one of the housing halves is shown);
Figures 34A, 34B and 34C are longitudinal cross-sectional views similar to Figure 31A, 31B, 31C showing initial distal movement of the handle portion (plunger) to move the advancer to the distal position to align the casing for the hole closure device and advance it into the sheath;
Figure 35 is a partially exploded view similar to Figure 33 corresponding to the position of the handle portion and advancer of Figures 34A-34C;
Figures 36A and 36B are longitudinal cross-sectional views similar to Figures 34A and 34B showing further distal movement of the handle portion to move the rails into the spaces in the housing;
Figure 37 is a partially exploded view similar to Figure 35 corresponding to the position of the handle portion and the advancer of FIGS. 36A and 36B;
Figure 38 is a partially exploded view similar to Figure 37 showing the handle portion fully advanced to the distal position;
Figures 39A, 39B and 39C are longitudinal cross-sectional views similar to Figures 34A, 34B and 34C showing full advancement of the handle portion to the distal position;
Figure 40 is a cross-sectional view taken along line 40-40 of Figure 39A; and
Figure 41 is a perspective view showing retraction of the delivery instrument after full advancement of the handle portion to further deploy the hole closure device.

### Description of embodiments

Referring now in detail to the drawings wherein like reference numerals identify similar or like components throughout the several views, the present application is directed to a delivery instrument for delivering a vascular hole (aperture) closure device. The closure device is intended to close an aperture in the vessel wall, typically formed after removal of a catheter previously inserted through the vessel wall into the vessel lumen for performing angioplasty or other interventional procedures. The aperture extends through the patient's skin and underlying tissue, through the external wall of the vessel, through the wall of the vessel, and through the internal wall of the vessel to communicate with the internal lumen of the vessel. The closure device of the present disclosure has an intravascular component to block blood flow and an extravascular component to retain the intravascular component.

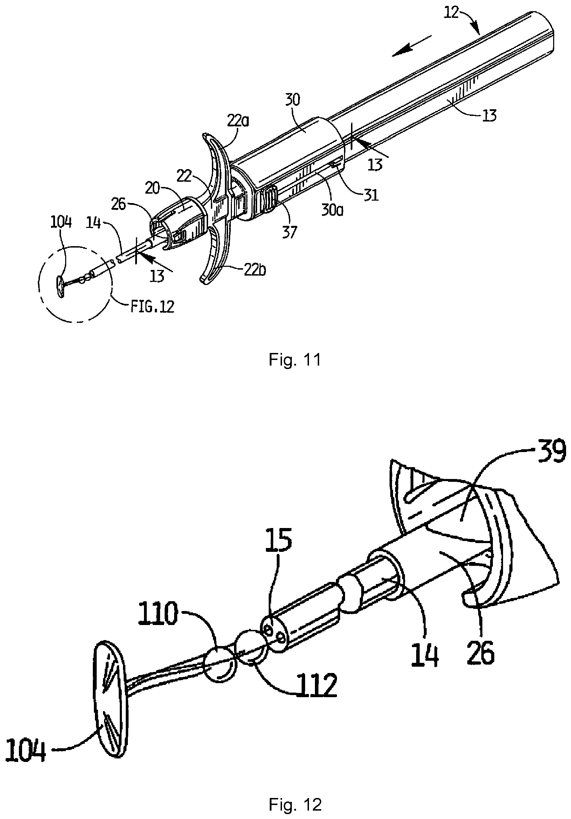

The hole closure device 101 is illustrated in Figures 12, 16, 18, 20 and 22 in various stages of delivery and is described in more detail in patent application 12/854,988, filed August 12, 2010, (hereinafter the '988 application). The closure device includes a covering member or patch 104 positioned within the vessel against the internal wall of the vessel to block blood flow through the vessel aperture and two retainers 110, 112 positioned external of the vessel wall to retain the covering member 104 in its blocking position. Each retainer 110, 112 is preferably spherical in configuration and is fixedly attached to a respective suture 122, 120, such that pulling of the respective suture advances the attached retainer toward the covering member 104 to ultimately position the retainers 110, 112 in a side by side relationship either against or adjacent the external surface of the vessel wall.

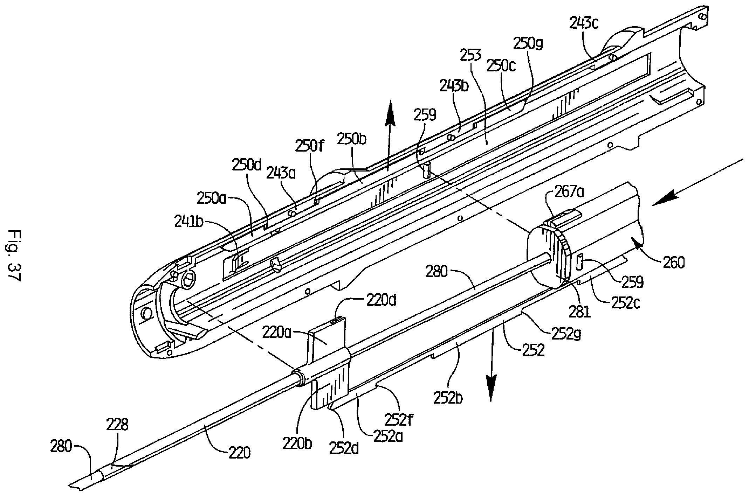

Covering member 104, preferably elongated in configuration as shown, is retained in a delivery sheath in a longitudinal position for delivery to the vessel, and then pivots to a transverse position within the vessel lumen (substantially perpendicular to an axis extending through the aperture) for orientation to cover (patch) the vessel aperture on the internal side. This movement is illustrated in Figures 37A-37D of U.S. Patent No. 7,662,161 (hereinafter the'161 patent).

The elongated covering member 104 functions to cover (patch) the internal opening in the vessel wall to prevent the egress of blood. The covering member 104 is preferably somewhat oval shaped with elongated substantially parallel side walls 106a, 106b, and end walls 108a, 108b connecting the side walls 106a, 106b. Other shapes of the covering member are also contemplated. The end walls 106a, 106b can have substantially straight wall portions, or curved wall portions. Covering member 104 preferably has a thicker region in the central region than the first and second end regions. Other dimensions and configurations are also contemplated.

The longitudinal axis of covering member 104 defines a lengthwise dimension and transverse axes define a shorter widthwise dimensions. The widthwise dimension of the covering member 104 is preferably, for a 6Fr device, in the range of about 2.5 mm to about 3.5 mm, and more preferably about 3.1 mm. Other dimensions are also contemplated. The width preferably is at least substantially equal to the dimension of the internal opening in the vessel wall to effectively cover the opening. In a preferred embodiment, the covering member 104 has a length in the range of about 7.5mm to about 9 mm (in a 6 French system), and preferably about 8 mm.

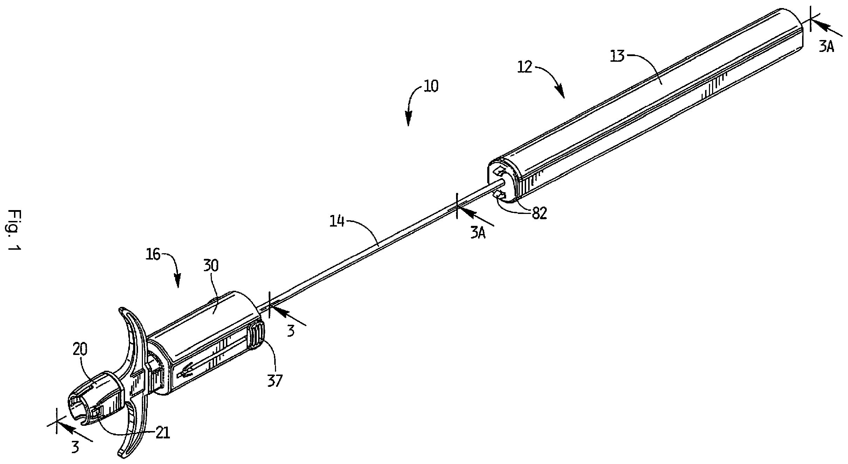

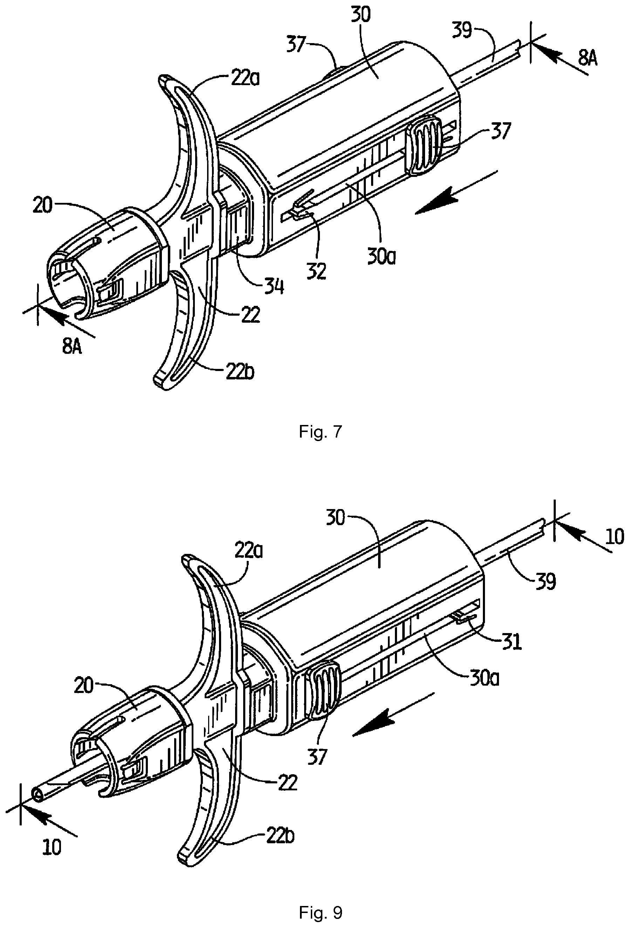

It should be appreciated that alternatively the covering member could be provided with an enlarged width region as illustrated in the embodiment of Figure 1 of the '161 patent. The covering member could also be configured asymmetrically so that the enlarged region is off-centered to accommodate widening of the aperture as the member is pulled at an angle. The covering member could also be configured in a paddle shaped with a narrowed region adjacent a wider region as in Figures 9B-9E of the '161 patent. Other covering member configurations including those disclosed in the '161 patent could be utilized with the retainers of this present application.

The elongated covering member can be composed of materials such as polycarbonate or polyurethane. Preferably it is composed of resorbable materials such as lactide/glycolide copolymers that after a period of time resorb in the body. If composed of resorbable material, the covering member could optionally have regions of varying resorbability. Varying degrees of resorbability can be achieved for example by utilizing different materials having differing resorbable characteristics or by varying the mass of the covering member (increased mass increases resorbtion time).

Spherical retainers 110, 112 are preferably composed of resorbable material. In a preferred embodiment, the diameter of each retainer 110, 112 is about 2.29mm (about .090 inches) to about 2.41mm (about .095 inches), although other dimensions are contemplated. Although shown as spheres, other shapes including other rounded shapes are also contemplated. The retainers could alternatively be made of non-absorbable polymeric or metallic material.

When the retainers 110, 112 are released from the delivery instrument, they are spaced further from the covering member 104. They are then configured to be advanced toward the covering member 104. More specifically, each retainer 110, 112 is fixedly secured to a respective flexible connecting member such as suture 122, 120. Sutures 122, 120 are preferably made of polymeric material and are preferably resorbable, composed of a material such as polydioxanome. It is also contemplated that alternatively a metallic material could be utilized. The sutures, retainers and covering member can be made of the same or different resorbable material, and/or have the same or different resorption times.

Details of the hole closure device as well as various embodiments of the device are shown and described in the '988 patent application previously incorporated by reference herein.

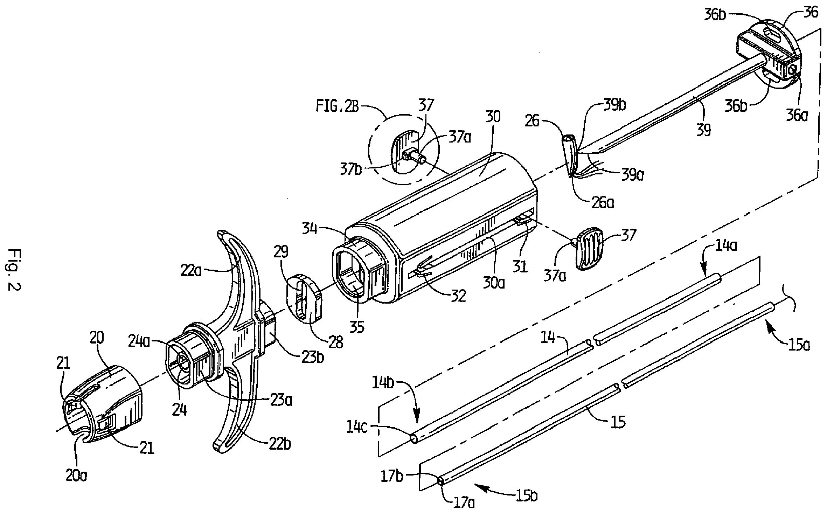

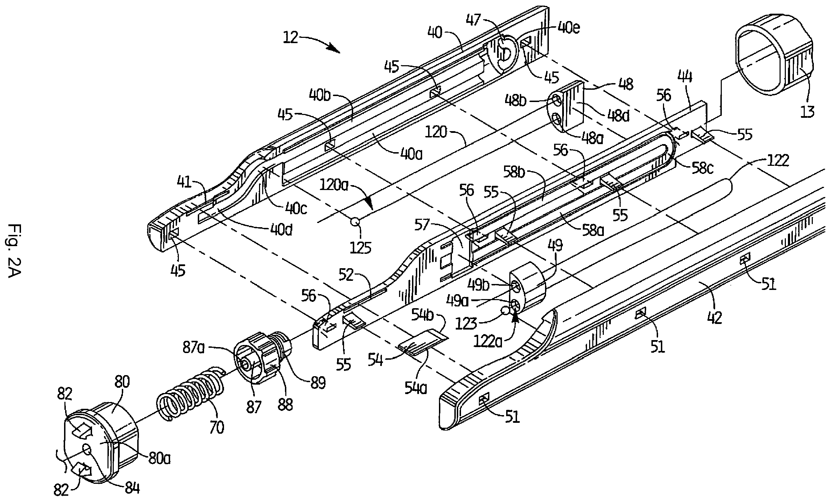

Suture 120 has a second end 120a (Fig. 2A) and a first opposite end secured to retainer 112 by molding, gluing, forming a knot, or other methods. Similarly, suture 122 has a second end 122a and a first opposite end secured to retainer 110 in any of the foregoing manners. Various methods of attachment are shown in the '988 application.

To advance the retainers 110, 112 toward the vessel wall (and covering member), the ends 122a, 120a of each suture 122, 120 is pulled proximally, thereby moving the respective retainer in the opposite direction closer to the aperture and vessel wall. This is described in detail below in conjunction with the delivery instrument. Note that once the retainers 110, 112 are tightened against the tissue, a sufficient retention force is maintained, i.e. a proximal pulling force on the covering member 104 to pull it slightly proximally against the vessel wall. The retainers 110, 112 therefore help to prevent the covering member 104 from separating from the vessel wall (e.g. moving in the direction toward the opposing vessel wall) which could create an unwanted gap between the covering member 104 and the vessel opening to allow blood flow. The extent to which the retainers 110, 112 move toward the wall (and thus their distance from the vessel wall in their final placement position) will depend on the tissue thickness. Thus, the closure device can adjust for different tissue thicknesses and apply a constant retention force regardless of tissue thickness.

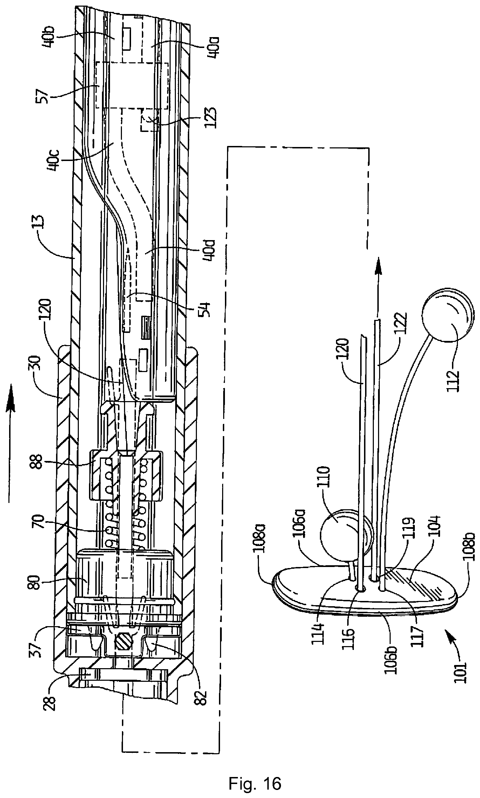

The covering member 104 as shown in Figure 16 has a first pair of holes and a second pair of holes. The first pair of holes 116, 117 receive suture 120 and the second pair of holes 119, 114 receive suture 122. Holes 114, 117 have a smaller diameter than holes 116, 119. The larger hole 116 is dimensioned to receive suture 120 for free unrestricted movement of the suture 120 therethrough and therefore easier application of spherical retainer 112. Similarly, the larger hole 119 is dimensioned to receive suture 122 for free unrestricted movement of the suture 122 therethrough and therefore for easier application (movement) of spherical retainer 110. Smaller hole 114 is dimensioned to frictionally engage suture 122 so that tension is applied to the suture 122. It is dimensioned so that the suture 122 can be pulled through the hole 114 if sufficient force is applied by pulling on second end 122a, but if such predetermined force is not applied, the suture will remain frictionally engaged within the wall of the opening 114 and not move. In this manner, when tension on end 122a is terminated, the suture 122 and thus the spherical retaining ball 110 will remain in position. Suture 120 operates in a similar manner, with smaller opening 117 dimensioned to frictionally engage and resist movement of the suture 120 to retain spherical retaining ball 112. Preferably, each hole 114, 117 has an inwardly angled wall transitioning into a reduced diameter region and an outwardly angled wall transitioning back to a larger diameter. The angled walls facilitate movement of the suture when tension is applied, with the reduced diameter region frictionally securing the suture. Hole 117 has a similar configuration as hole 114 and thus also contains similar angled walls. In this manner, when tension on end 120a is terminated, the suture 120 and thus the spherical retaining ball 112 will remain in position.

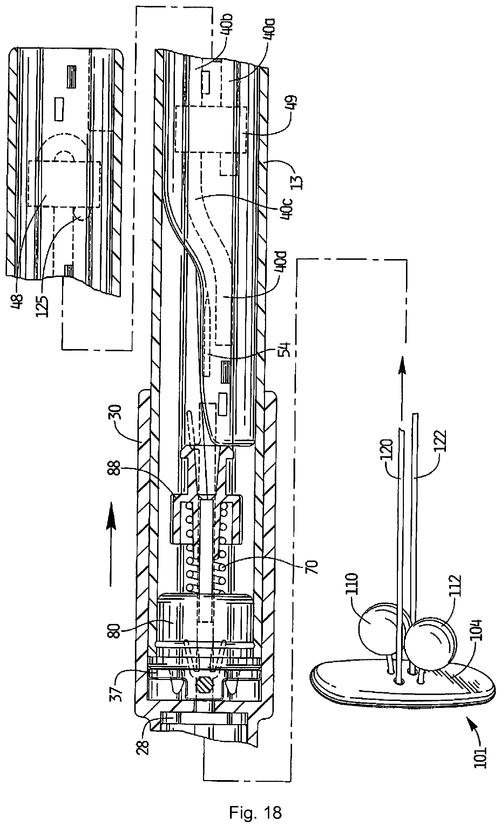

As shown in Figures 16 and 18, retainer 110 is pulled towards the smaller hole 114 and retainer 112 is pulled toward the smaller hole 117. However, it is also contemplated, to facilitate manufacture, that the sutures could be reversed so the retainer 110 is pulled toward the larger hole 116 and the retainer 112 is pulled toward the larger hole 119.

A crimp or a bead can be attached to the suture, or a knot formed in the suture, creating a diameter larger than the diameter of portion within the retainer which forms a shoulder to block movement of the respective spherical retainer 110 or 112. Consequently, this frictional engagement prevents the respective retainer from sliding in the direction away from the covering member 104 while the shoulder prevents the retainer from sliding in the direction toward the covering member 104. The retainer 112 and suture 120 preferably have the same structure and engagement/retention as retainer 110 and suture 122.

Note that during delivery the covering member 104 emerges from the delivery sheath and moves from a tilted position, more aligned or in preferred embodiments substantially aligned with the longitudinal axis of the sheath, to a transverse position within the vessel. This is due to a preset in the sutures which maintains the covering member in the transverse position.

As can be appreciated, covering member 104 is pulled proximally to abut the internal opening on the internal side of the vessel to cover (patch) the opening and the sutures extend through the opening in the vessel wall. Note that in the delivery position, the retainers 110 and 112 are preferably in a stacked relationship within the delivery instrument to minimize the transverse dimension of the delivery system.

Then, to retain the covering member 104 in position against the vessel wall to block blood flow therethrough, sutures 120, 122 are pulled proximally from their ends 120a, 122a, thereby advancing the retainers 112, 110 toward the vessel wall and covering member 104. The retainers 112, 110 can be moved to a position contiguous to the vessel wall, or depending on tissue thickness, may be adjacent the wall with some tissue interposed between the retainers and vessel wall. The retainers 110, 112 in this position apply a proximal force on the elongated covering member 104 to limit movement of the covering member into the vessel. The retainers in this placement position are preferably in a substantially side by side relationship. The instrument for delivering these elements to the target site to close the vessel opening is described in detail below.

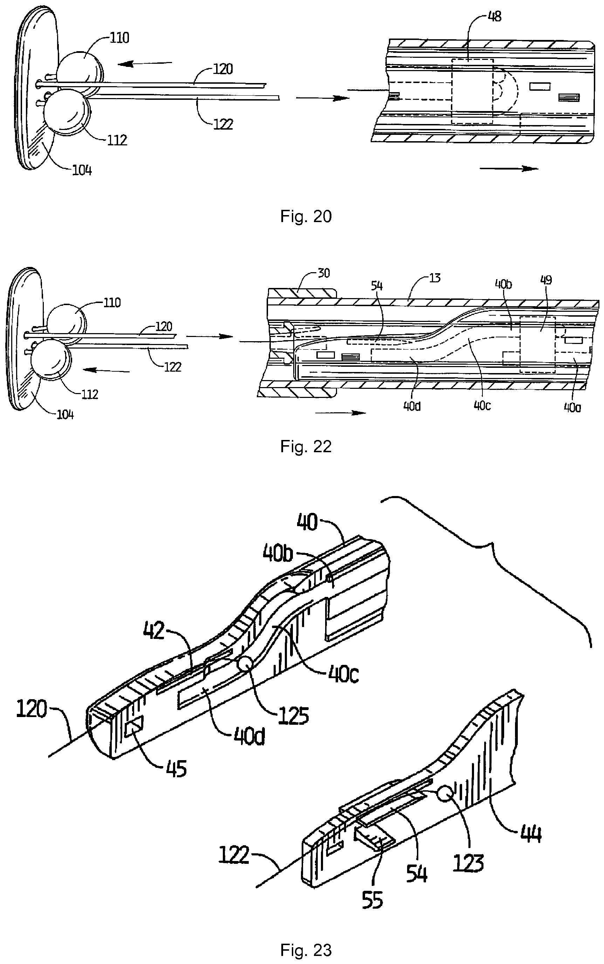

As shown in Figure 22, in the side by side relationship, the retainers 110, 112 are alongside in a transverse orientation with respect to covering member 104. That is, they are positioned along the width of the covering member 104 However it is also contemplated that the retainers in the placement position can be in a lengthwise orientation (substantially parallel to the longitudinal axis of the covering member.) The retainers could also be in other side by side arrangements at angles to the longitudinal axis. Alternatively, the retainers can be partially stacked in the placement position.

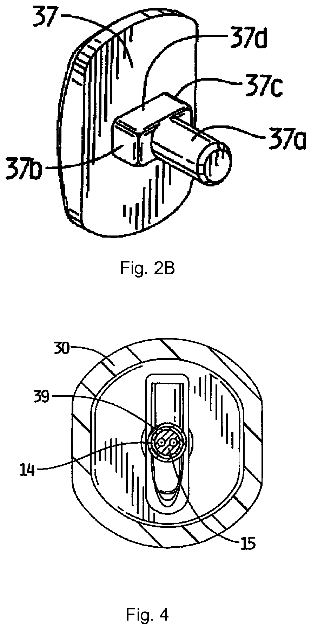

Turning now to the delivery instrument of the present disclosure and with initial reference to Figures 1 and 2, the delivery instrument is designated generally by reference numeral 10 and includes a handle portion 12, an elongated outer tube 14 extending distally from the handle portion 12, and an advancer portion 16. Handle portion 12, as shown in Figure 2A, includes a first housing half 40, a second housing half 42 and a central housing support 44 which together form channels for passage of the sutures. Further details of the handle portion (plunger) 12 are described in detail below.

The delivery instrument 10 for inserting the closure device extends through an opening in the patient's skin, through the underlying tissue, through an external opening in the vessel wall, through the aperture in the vessel wall, and through an internal opening on the internal side of the vessel wall into the vessel lumen.

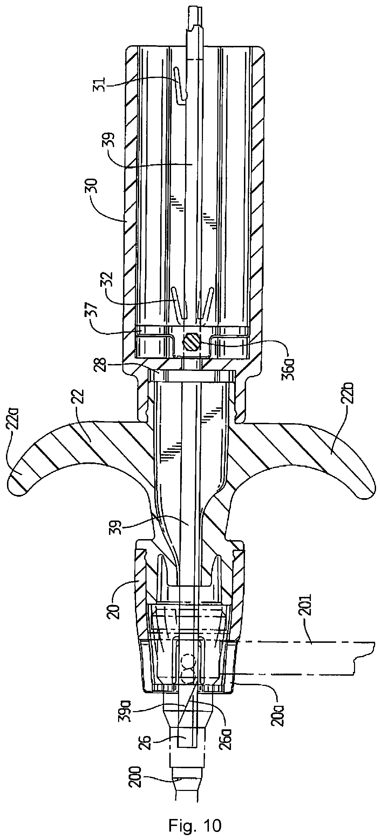

The delivery instrument 10 deploys the hole closure device 101 in a multiple step process. In the first step, the advancer portion 16 reorients the hole closure device 101 and advances the hole closure device from the delivery instrument 10, and into a proximal end of a sheath (Fig. 10). In the second step, the handle portion 12 is advanced with respect to the advancer portion 16 to advance the hole closure device 101 through the sheath 200 and into the vessel lumen of a patient. In the third step, the entire delivery instrument 10 is retracted proximally to move the retainers 110, 112 toward the covering member 104. This multiple step process is described in detail below.

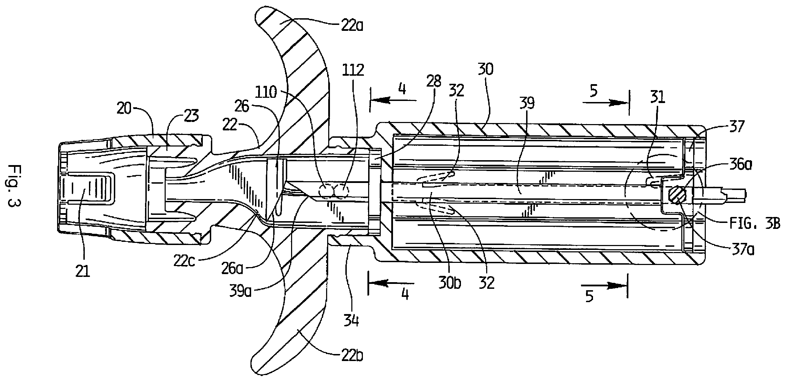

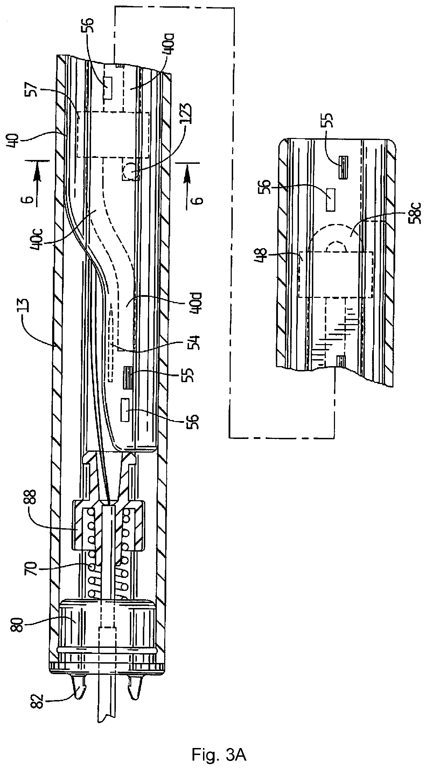

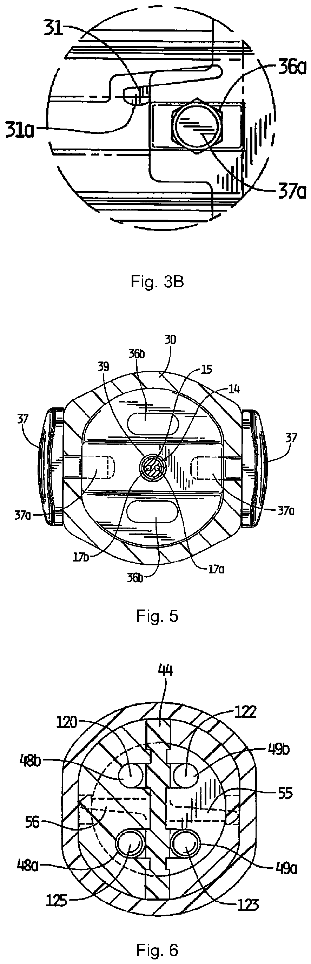

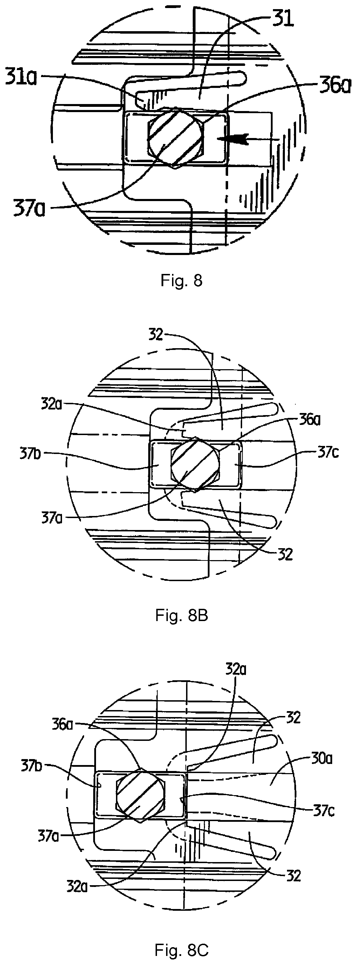

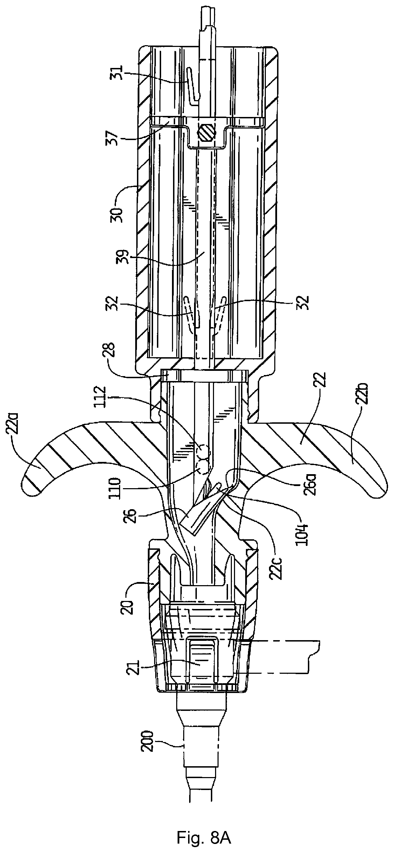

Turning first to the advancer portion 16 of the delivery instrument, and with initial reference to Figures 2 and 3, advancer portion 16 includes a winged housing 22, a guide housing 30 and an advancer tube 39. Advancer tube 39 is slidably positioned within a lumen in guide housing 30. Winged housing 22 has radially extending wings 22a, 22b to be gripped by the user during use, e.g. to aid in retraction of the delivery instrument 10 after the hole closure device 101 is deployed in the vessel. Distal end 23a of winged housing 22 is dimensioned to receive cap 20 thereover, preferably connected by an interference fit, and proximal end 23b is dimensioned to be received within opening 35 of connector portion 34 of guide housing 30, also preferably providing an interference fit. Winged housing 22 can also be connected to connector portion 34 of housing 30 by groove/projection snap fit, a threaded engagement or any other method of attachment. Similarly, cap 20 and winged housing 22 can also be connected by groove/projection snap fit, a threaded engagement or any other method of attachment. Cap 20 has a pair of tabs 21, preferably formed by cutouts in the housing, to engage a sheath 200 as shown in Figure 8A to connect the sheath to the winged housing 22. Opening 20a in cap 20 allows for a side tube of the sheath 200, the side tube enabling fluid injection through the sheath 200.

Seal 28 is supported within an opening 35 of connector portion 34 of housing 30 to prevent ingress of fluids. Seal 28 has an opening 29 dimensioned and configured to receive advancer tube 39 therethrough.

Advancer tube 39 forms a cheater tube slidable within winged housing 22 and guide housing 30. Advancer tube 39 is in the form of a tube cut at the distal end to form a hinge 39b (Fig. 2). The cut portion forms a casing 26. Casing 26 is somewhat cylindrical shaped with a beveled end 26a formed by the cut in the tube. Casing 26 forms a support housing for the covering member 104 of hole closure device 101. The casing 26 is initially mounted within the lumen of winged housing 22 in a position substantially transverse to a longitudinal axis of the winged housing 22 as shown in Figure 3. Distal movement of the advancer tube 39 pivots the casing 26 (Figure 8A) about hinge 39b at it contacts curved wall 22c within winged housing 22 to a more linear position substantially aligned with the longitudinal axis of the winged housing 22 as shown in Figure 10. This allows for pivoting movement of the elongated covering member 104 from a transverse position to reposition it to a more aligned position for advancement through the sheath 200. The deployment of the closure device 101 is described in more detail below. Casing 28 is pivoted and moved within the tubular channel 24 of winged housing 22, exiting opening 249. Note also that in the substantially aligned position of casing 26 of Figure 10, the beveled distal end 39a of advancer tube 39 abuts the beveled end 26a of casing 26.

Guide housing 30 of advancer portion 16 has a pair of slots 30a formed in the outer wall and on opposing sides thereof. Slots 30a extend longitudinally along the housing 30 and are each configured and dimensioned to receive a sliding finger tab 37. More specifically and with reference to Figures 2 and 2B, each sliding tab 37 has a post 37a extending radially inwardly toward the longitudinal axis of the housing 30. Each post 37a extends through an opening 36a (preferably hexagonal although other shapes are contemplated) in proximal end cap 36 of advancer tube 39. In this manner, movement of the sliding tabs 37 within slots 30a causes sliding movement of the advancer tube 39 through guide housing 30. This is shown in Figures 7-10, wherein in Figures 7 and 8A, sliding tabs 37 have been moved slightly distally from their initial position of Figure 1 causing slight distal movement of the advancer tube 39 from its initial position. As can be appreciated, in this position, casing 26 has begun to be pivoted about hinge 39b at the distal end of advancer tube 39. In Figures 9 and 10, sliding tabs 37 have been moved further distally to their distal position, causing further distal movement of advancer tube 39 to its distal position. This causes full rotation of casing 26 to its aligned position and movement of casing 26 into a proximal region of sheath 200.

Cap 36 of advancer tube 39 has a pair of openings 36b on opposing sides to receive tabs 82 of cap housing 80 (Figure 2A) of handle portion 12 discussed below.

In the initial proximal position of sliding tabs 37, a stop 31 limits their distal movement. Stop 31 in is the form of a finger formed by a cutout in housing 30, and can be provided for each tab 37, or alternatively a single stop can be provided to engage one of the sliding tabs 37. The finger 31 projects into slot 30a providing a bump 31a to block movement of tab 37 as flat 37b (Fig 2B) on the distal side of block 37d supporting post 37 abuts finger 31. That is, as flat 37b contacts finger (stop) 31, finger 31 prevents inadvertent movement of the sliding tabs 37, such as during shipping. In order to distally advance the tabs 37, sufficient force must be applied to override the stop 31, e.g. flex the finger 31 out of the path of the block 37d so block 37d of tab 37 can continue along slot 30a.

Lockout 32 is positioned distal of stop 31 and locks sliding tabs 37 in their distal position. A lockout 32 can be provided on each side of the housing 30 (at the distal end of each slot 30a) or alternatively only one lockout can be provided to lock one of the sliding tabs 37 which would effectively lock both tabs 37. More specifically, the lockout is in the form of two fingers 32 formed by cutouts in housing 30 which project into slot 30a, thereby narrowing the slot 30a at region 30b (Figure 3). Upon distal movement of tabs 37, flat 37b forces fingers 32 out of the path to move past the fingers 32 as shown in Figures 8B and 8C. Flat 37c on the opposing side of flat 37b then engages edge 32a of fingers 32 to prevent retraction of tab 37, thereby locking tabs 37 and thus advancer tube 39 in the forward (distal) position.

Referring back to Figure 2, an inner tube 15 is concentrically mounted within lumen 14c of outer tube 14. Outer tube 14 has a proximal end 14a extending from the handle portion 12 and connected within opening 84 to cap 80 (Figure 2A) of handle portion 12. A distal end 14b of outer tube 14 extends into the guide housing 30 and is configured to abut the retainer of the hole closure device 101 to advance it through the sheath 200. Inner tube 15 has a proximal end 15a and a distal end 15b, with proximal end 15a connected to connector 88 (Figure 2A). Inner tube 15 has first and second channels 17a, 17b to receive sutures 120, 122.

Turning now to the handle portion 12 which provides the second step in advancement of the hole closure device 101, and with initial reference to Figures 2A and 3A, handle portion 12 includes a handle housing (outer casing) 13 containing a central housing 44 and first and second channel housing halves 40, 42, respectively, which are mirror images of each other. Central housing 44 is mounted between housing halves 40 and 42. Each of the housing halves 40, 42, 44 taper to a reduced diameter portion at the distal end.

Channel housing half 40 has a plurality of axially spaced openings 45 to receive radially extending tabs 56 of central housing 44. Similarly, channel housing half 42 has a plurality of axially spaced openings 51 to receive radially extending tabs 55 of central housing 44. This tab/opening engagement connects the housings 40, 42 and 44 together.

First channel housing 40 together with a first side of central housing 44 (e.g. the left side as viewed in Fig. 2A) together from a channel for passage of the suture 120 and engagement member 125 secured to the suture 120 in order to advance the retainer 112 toward the covering member 104 of hole closure device 101. Second channel housing 42 together with a second side of central housing 44 (e.g. the right side as viewed in Fig. 2A) together from a channel for passage of the suture 122 and engagement member 123 secured to the suture 122 in order to advance the retainer 110 toward the covering member 104 of hole closure device 101.

With continued reference to Figure 2A, housing 40 has a lower channel 40a, an upper channel 40b, and a curved channel 40e joining channels 40a, 40b at their proximal end. At the distal end of upper channel 40b, the upper channel 40b transitions into an angled channel 40c and then into a longitudinally extending channel 40d extending substantially parallel to the longitudinal axis of the housing 40. The angled channel 40c facilitates severing of the suture 120 discussed in more detail below. Identical channel portions are on housing half 42. Thus, housing half 42 has an upper channel, a lower channel, a curved channel joining the upper and lower channels at a proximal end, an angled channel and a longitudinally extending channel.

The central support 44 has a channel on each of its sides to cooperate with the channel in the housing halves 40, 42, thereby together forming the channel (passage) for the respective suture 120, 122 and engagement member 125, 123. This can be best understood in Figure 2A wherein central housing 44 has a lower channel 58a, an upper channel 58b and a curved channel 58c at the proximal end joining lower channel 58a and upper channel 58b. Central support 44 has the identical channel configuration on the opposing side, except as noted below. Thus, on the opposing side, the upper, lower and curved channels of central housing 44 cooperate with the channels 40a, 40b and 40e of first housing 42. On the side shown in Figure 2A, the channels 58a, 58b and 58c of housing 44 cooperate with the upper, lower and curved channels of housing 42.

The only difference in the channels on opposing sides of central housing 44 is the recess to receive the respective grommet 48, 49. More specifically, central housing 44 has a distal recess or groove 57 cooperating with a recess on housing 42 for mounting of distal grommet 49. A proximal recess or groove is formed on the opposing side of central housing 44, cooperating with the proximal recess 47 of housing 40 to provide a recess or groove to receive proximal grommet 48.

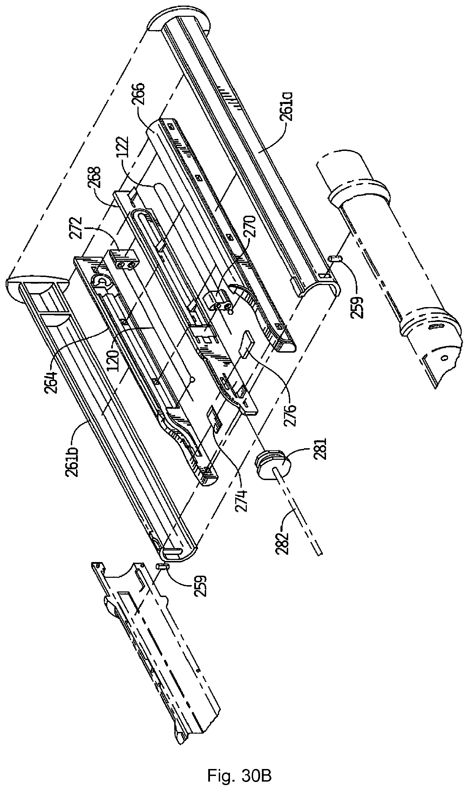

Distal grommet 49 is illustratively semi-circular in cross-section and has a flat surface for positioning within recess 57 of central housing 44. Other geometries are also contemplated to key the grommets such as the asymmetric configuration shown in Figure 30B for example. Grommet 49 forms an abutment member or blocking member for engagement member 123 attached to the end of suture 122. Grommet 49 has a lower opening 49a aligned with lower channel 58a (and corresponding lower channel on housing half 42) and an upper opening 49b aligned with upper channel 58b (and corresponding upper channel on housing half 42). Preferably, upper opening 49b is smaller than lower opening 49a to provide an increased frictional force for passage of engagement member 123 therethrough.

Proximal grommet 48 is illustratively semi-circular in cross-section and has a flat surface 48d for positioning within a recess of central housing 44. As with grommet 49, other geometries are also contemplated, such as that shown in Figure 30B. Grommet 48 forms an abutment or blocking member for engagement member 125 attached to the end of suture 120. Grommet 48 has a lower opening 48a aligned with lower channel 40a (and corresponding lower channel on central housing 44) and an upper opening 48b aligned with upper channel 40b (and corresponding upper channel on central housing 44). Preferably upper opening 48b is smaller than lower opening 48a to provide an increased frictional force for passage of engagement member 125 therethrough.

An elongated knife slot 41 is formed in housing 40 and an identical knife slot is formed in housing 42. These knife slots extend substantially parallel to a longitudinal axis of the housings 40, 42. The knife slots are aligned with knife slot 52 in central housing 44 to receive knife 54. Knife 54 has a substantially planar surface 54a and a proximally directed sharp edge 54b to sever sutures 120, 122 as described in detail below.

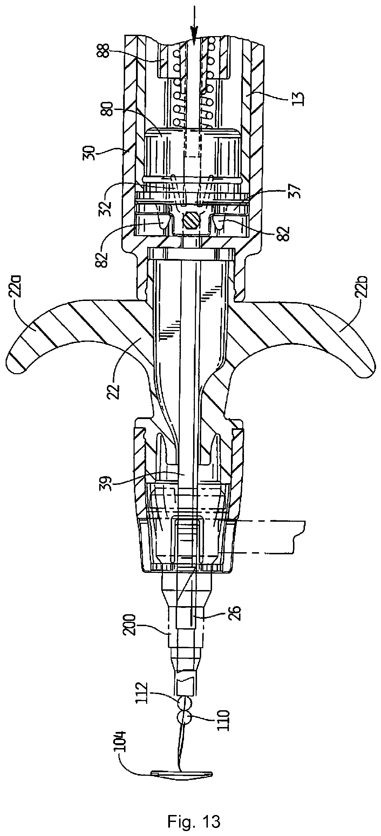

At the distal end of handle housing (casing) 13 is a cap 80 having a central opening 84 to receive outer tube 14 and concentric inner tube 15. A pair of finger tabs 82 extending distally from a distal surface 80a snap into openings 36b of cap 36 of advancer tube 39 (Fig. 2) when the handle housing 13 is advanced to its distalmost position (see e.g. Fig. 13). Connector 88 has an opening 87a in tubular portion 87 to receive outer tube 14 and concentric inner tube 15. A spring 70 can also be provided to act as a spacer.

After the advancer tube 39 is advanced by tabs 37 in the manner described above, the handle housing 13 is advanced distally, preferably in increments, causing outer tube 14 to contact the proximally positioned retainer 112 of hole closure device 101 and advance it through the sheath 200 and out the distal end thereof. This is shown in Figure 11, wherein the sheath has been removed for clarity. The handle housing 13 is advanced until tabs 82 of cap 80 lockingly engage advancer tube cap 36 as the tabs 82 extend through openings 36b and snap fit therein as shown in Figure 13. After such full advancement of handle housing 13, the covering member 104 and retainers 110, 112 are positioned in the vessel, with the retainer 104 moving to a transverse position due to the pre-set of the sutures 120, 122. That is, the sutures 120, 122 are preset at an angle of about 90 degrees such that when the covering member 104 is free of the confines of the sheath 200, it automatically pivots to the transverse position of Figures 11 and 12.

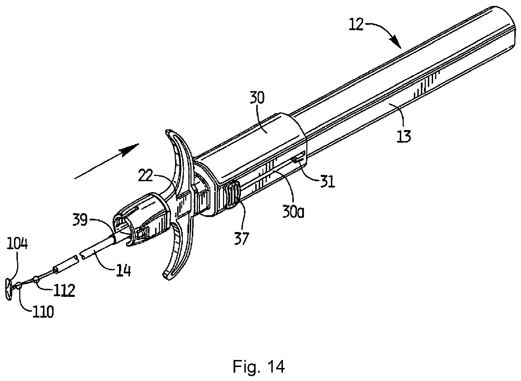

Once the handle housing 13 is advanced and locked to the advancer portion 16, the delivery instrument 10 is then retracted in the direction of the arrow of Figure 14. Retraction of the handle housing 13 moves the sutures 120, 122 and engagement members 125, 123 through the channels in the housings 40, 42, 44 as described in detail below. Thus, as can be appreciated, in use, in this multiple step process, first the advancer tube 39 is advanced to rotate the casing 26 to the position shown in Figure 10 and to move it into the proximal region of the sheath 200. Next, the handle housing 13 is advanced relative to the fixed (locked) advancer portion 16 to advance the hole closure device 101 through the sheath 200 and out the distal end thereof. Once the handle housing 13 has completed its distal travel and interlocked with advancer portion 16, the handle housing 13 (and the entire delivery instrument 10) is retracted thereby securing the patch 104 by movement of the retainers 110, 112 in sequence towards the covering member 104.

The engagement members 123, 125 move within the channels and interact with the grommets 48, 49. The grommets 48, 49 have openings providing engaging portions or abutment or blocking members which provide resistance to movement of the sutures 120, 122. This resistance is achieved by the provision of engagement members 123, 125 on the ends of suture 120 and suture 122. More specifically, an engagement member 125, illustratively substantially spherical in configuration, although other shapes are contemplated, is positioned at the end 120a of suture 120. Similarly, an engagement member 123, illustratively substantially spherical, although other shapes are contemplated, is positioned at the end 122a of suture 122. Engagement members 125, 123 can be attached by methods such as crimping, tying a knot, overmolding, etc. and are configured to engage respective grommets 48, 49 to provide resistance to suture movement.

The use of the delivery device 10 to deliver hole closure device 101 will now be described. Note delivery instrument is connected to a delivery sheath 200. The delivery sheath 200 is inserted through the skin, the tissue puncture tract extending to the vessel wall, and through the vessel wall into the vessel lumen. In the initial position, the retainers 110 and 112 are positioned within advancer tube 39 within winged housing 22 as shown in Figure 3. Covering member 104 is maintained in a transverse position within casing 26. In this initial position, engagement members 125 and 123 of sutures 120, 122, respectively, are out of engagement with the respective grommets 48, 49 of the handle housing 13.

To deploy the closure device 100, the advancer tube 39 is advanced distally by finger tabs 37 to pivot casing 36 from the initial position of Figure 3, through the position of Figure 8A, to the position of Figure 10 (due to curved wall 22c), thereby aligning the covering member 104 longitudinally for passage through the winged housing 22 and sheath 200, and moving the casing 36 into the proximal region of the sheath 200. Note the tabs 37 of advancer portion 16 are locked in their distal position by locking fingers 32 of guide housing 30.

Next, handle housing 13 is moved distally relative to the fixed advancer portion 16 such that outer tube 14 advances through fixed advancer tube 39 to contact retainer 112 to advance the hole closure device 101 through the sheath 200 and out the distal end of the sheath as shown in Figure 13 (see also Figures 11 and 12). Note that handle housing 13 in its distal position interlocks with the advancer portion 16 due to the engagement of tabs 82 of cap 80 with openings 36b in advancer tube end cap 36. Once the covering member 104 is exposed, it pivots within the vessel lumen from a first delivery position more aligned with the longitudinal axis of the delivery sheath to a transverse placement position as shown in Figures 11-13.

The delivery instrument 10 is then retracted proximally in the direction of the arrow of Figure 14 to place the covering member 104 against the internal side of the opening in the vessel wall to patch or cover the vessel wall opening. Figures 11 and 12 show the initial position of the retainers 110, 112 when the closure device 101 is initially inserted into the vessel lumen.

When the delivery instrument 10 is retracted further such that the covering member 104 abuts the internal vessel wall, further retraction of the delivery instrument 10 will deploy the retainers 110, 112 as follows. In the initial position of Figure 3, engagement member 125 is within lower channel 40a of housing 40 (Figure 2A) and engagement member 123 is in lower channel 58a of central housing 44 slightly distal of lower opening 49a of proximal grommet 49 (Figure 3A). It should be appreciated that as discussed herein, the channels of housing 42 cooperate with the channels on one side of central housing 44 and the channels of housing 40 cooperate with the channels on the opposing side of central housing 44. Thus, for brevity, only one of the cooperating channels is mentioned, it being understood that the channels are formed by cooperation of these components.

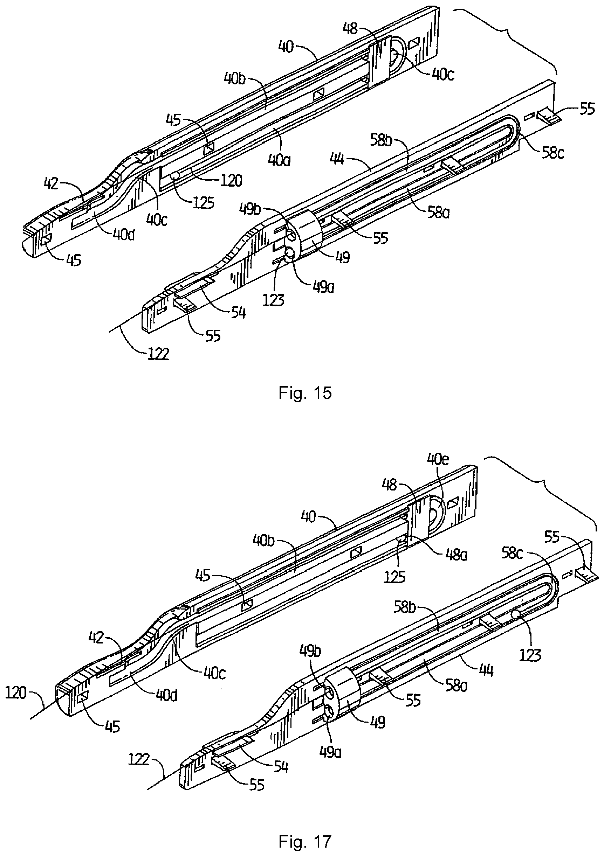

Upon further proximal retraction of the handle housing 13, suture 122 is pulled proximally such that engagement member 123 of suture 122 is pulled against an engaging portion of grommet 49, i.e., opening 49a in grommet 49, as shown in Figures 15 and 16. (Note the force of covering member 104 against the vessel wall provides a counter force such that proximal movement of the delivery instrument 10 and sutures 120, 122 cause distal movement of the retainers 110, 112 attached to the sutures 122, 120). The pulling (tensioning) of the suture 122 causes retainer 110, attached to the opposing end of suture 122, to move toward the covering member 104 as shown in Figure 16. Note that the engagement member 125 of suture 120 is not yet engaged with proximal grommet 48. In this position, distal grommet 49 provides a stop to restrict movement of the suture 122. This engagement also provides a tactile feel to the user to indicate that retainer 110 has moved a substantial distance toward covering member 104.

When delivery instrument 10 is pulled further proximally with respect to the delivery sheath 200 it pulls (tensions) suture 120 proximally to move retainer 112 toward covering member 104 as shown in Figure 18. Such movement continues until engagement member 125 abuts/engages an engaging portion of proximal grommet 48, i.e., at lower opening 48a of proximal grommet 48, as shown in Figures 17 and 18. Grommet 48 thereby provides a stop to limit movement of the suture 120. Grommet 48 also provides a tactile feel to the user to indicate that retainer 112 has moved a substantial distance toward covering member 104. Note that engagement member 123 has already overcome distal grommet 49, and passed through its lower opening 49a, and is no longer in tension. As can be appreciated, retainers 110 and 112 have now been moved adjacent the covering member 104 but not yet in their fully distal securement position. Note this delivery method distributes the force, e.g. reduces the load on the patch.

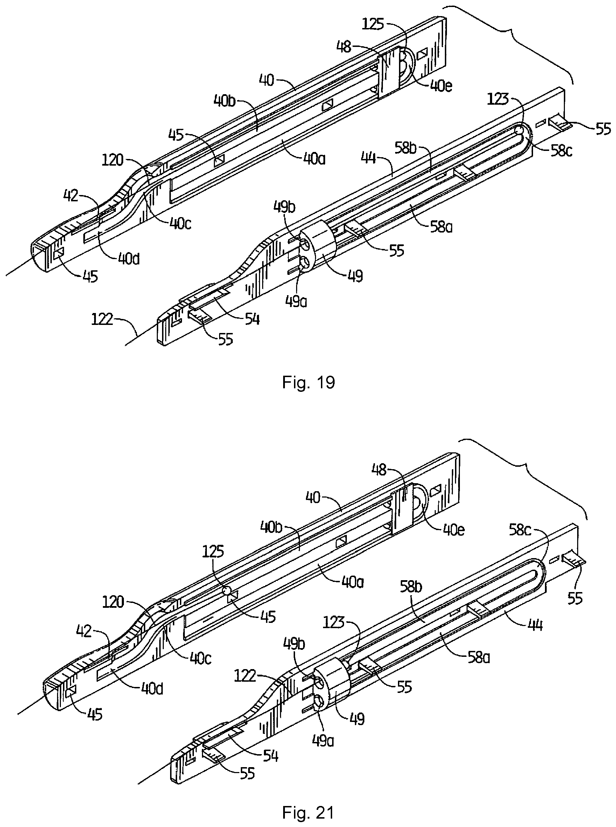

Continued proximal movement of delivery instrument 10 applies sufficient tension on suture 120 so engagement member 125 passes through lower opening 48a of grommet 48 and continues its travel around curved channel 40e until it engages grommet 48 at upper opening 48b. This position is shown in Figures 19 and 20. This moves retainer 112 further distally toward covering member 104 to tighten retainer 112 with respect to covering member 104. Note engagement member 123 continues its movement along upper channel 58b (after passing around proximal curved channel 58c) toward upper opening 49b of distal grommet 49.

Continued proximal movement as shown in Figures 21 and 22 pulls suture 122 proximally, moving engagement member 123 into engagement with upper opening 49b. Engagement member 125 has overcome engagement with proximal grommet 48. Further movement of suture 122 moves retainer 110 further distally toward covering member 14, thereby tightening retainer 110 with respect to covering member 104, securing the covering member 104 in position. Note the extent of movement of the retainers 110, 112 toward the covering member 104, i.e. the final distance between the retainers 110 and 112 and covering member 104, will depend on the thickness of the patient's tissue.

With placement of the retainers 110 and 112 within the tissue tract leading to the vessel opening (but outside the vessel opening), the sutures 122, 120 are now severed automatically by the cutting blade 54 of delivery instrument 10. This is illustrated in Figures 23-26.

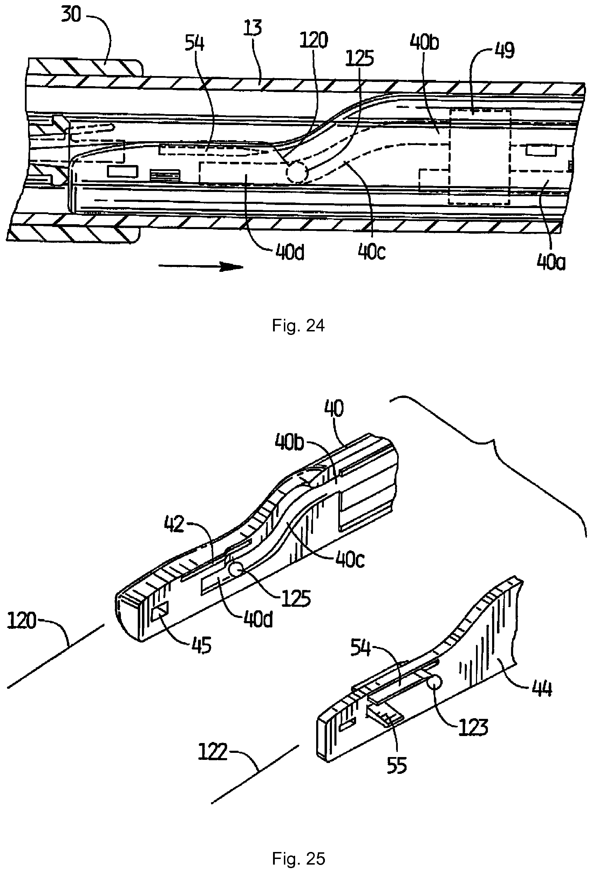

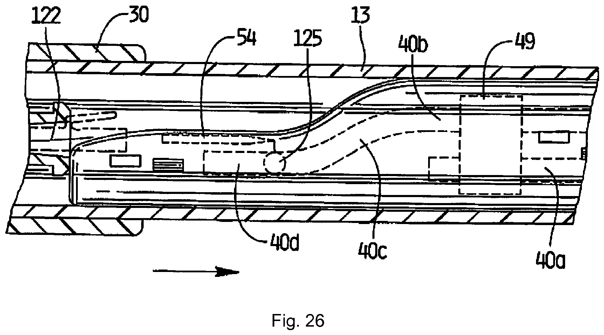

As the delivery instrument 10 is pulled further proximally, engagement members 125, 123 and sutures 120, 122 enter angled channel 40c on channel housing half 40 and an identical angled channel on the opposing channel housing half 42. As delivery instrument 10 is retracted further and sutures 120 and 122 are retracted further, engagement members 123, 125 move within longitudinal channel 40d on housing 40 (Figures 23 and 24) and an identical longitudinal channel on housing 42, with the sutures 120, 122 remaining above these longitudinal channels to contact cutting edge 54a of knife 54 (Figures 25 and 26) to sever the sutures 120, 122 as shown in Figure 26. Note engagement members 123, 125 can float inside the channel because they are no longer in tension. The sutures 120, 122 can be further tightened and then trimmed by the surgeon to be flush with the patient's skin.

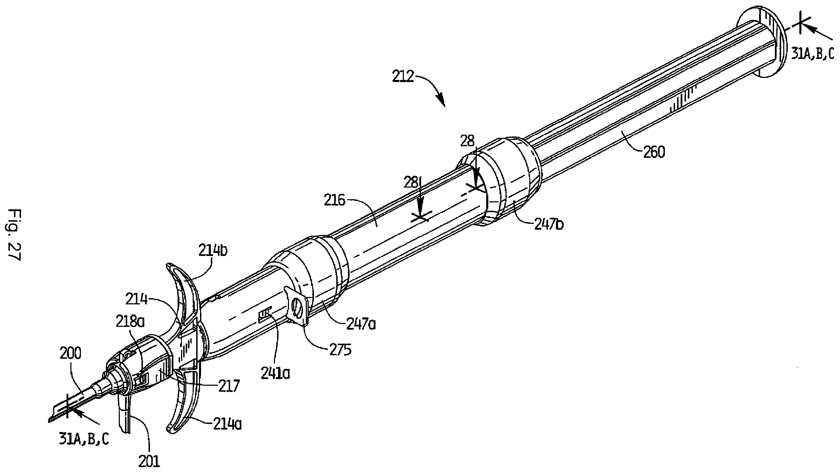

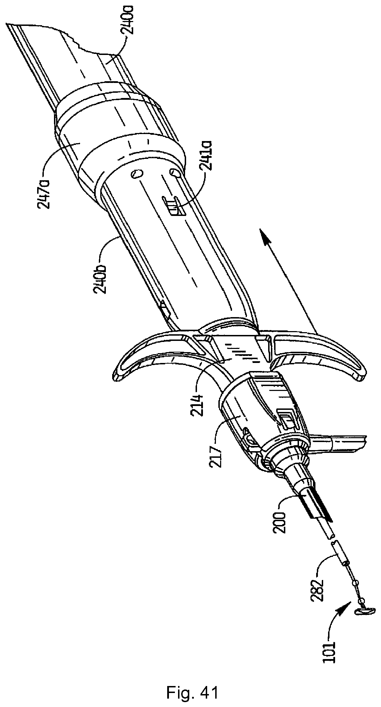

An alternate embodiment of the hole closure delivery instrument is illustrated in Figures 27-41. In this embodiment, the handle portion performs the multiple steps of advancing the advancer (cheater) tube to rotate the hinged casing at the end of advancer tube containing the covering member 104 of the hole closure device 101 as well as advances the hole closure device 101 through the sheath for delivery to the vessel. After delivery, the instrument is retracted and the retainers 110, 112 are advanced toward the covering member 104 in the same way as in the embodiment of Figures 1-26.

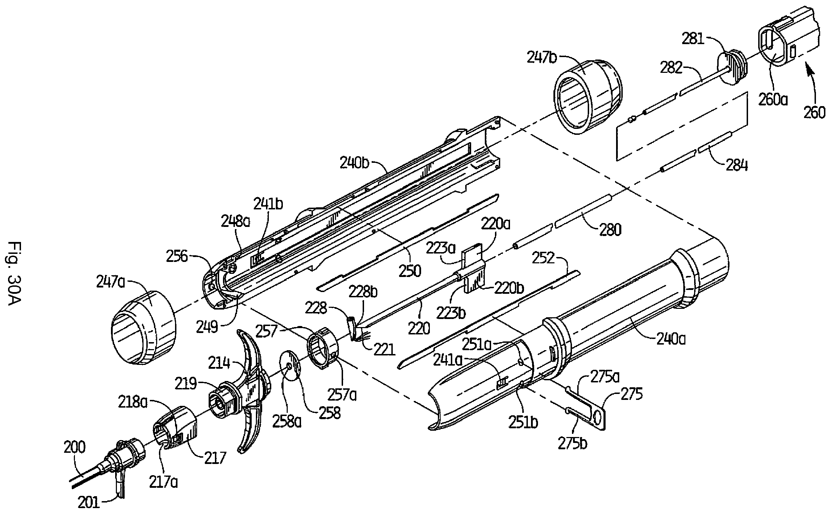

Turning first to Figures 27 and 30A, delivery instrument is designated generally by reference numeral 212 and has a winged housing 214, a plunger guide housing 216 formed by housing halves 240a, 240b and a handle portion or pusher (plunger) 260. Plunger 260 is slidably received within guide housing 216.

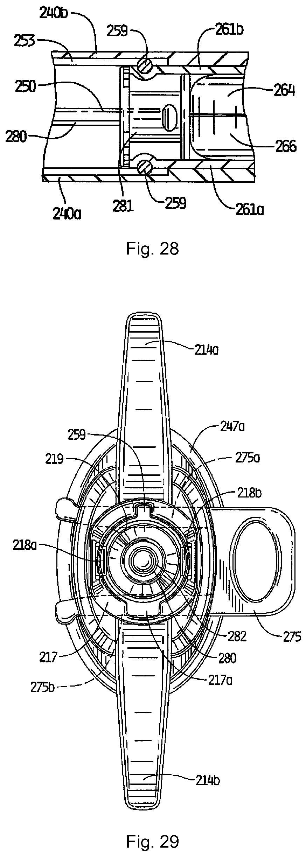

Winged housing 214 is connected to end cap 217, preferably by an interference or snap fit as distal end 219 is dimensioned to receive end cap 217 thereover. End cap 217 has locking tabs 218a, 218b (Figure 29) on opposing sides to engage sheath 200 in the same manner as locking tabs 21 of end cap 20 of the embodiment of Figure 1.

Winged housing 214 has a pair of wings 214a, 214b extending radially therefrom for grasping by the user to facilitate movement of the plunger 260 and to aid in retraction of the delivery instrument 212 after the hole closure device 101 is delivered to the vessel lumen. Cap 217 has an opening 217a through which side arm 201 of sheath 200 can extend as in opening 20a of end cap 20. Side arm 201 provides for delivery of fluids through the sheath 200.

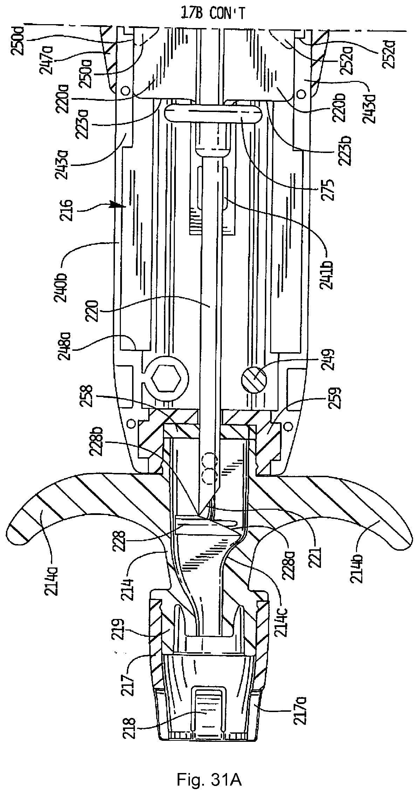

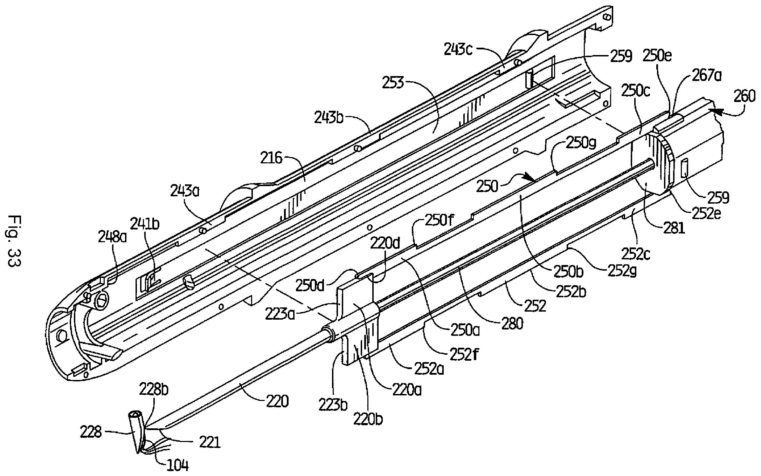

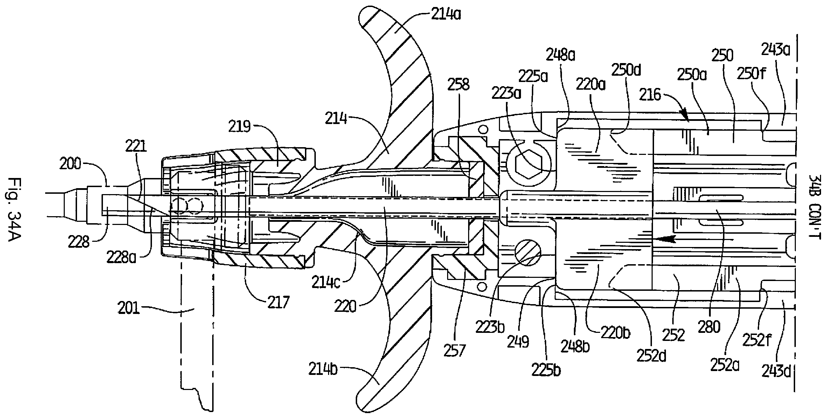

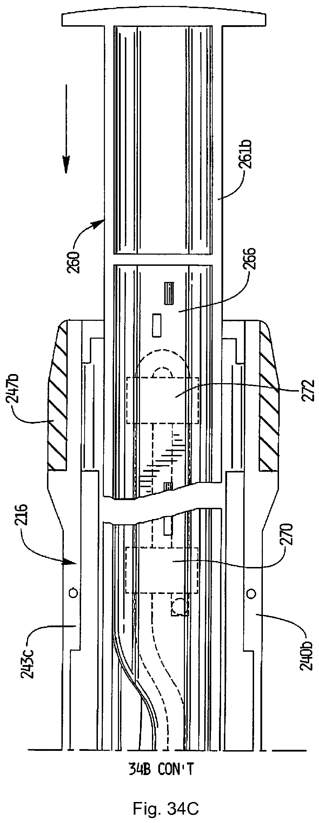

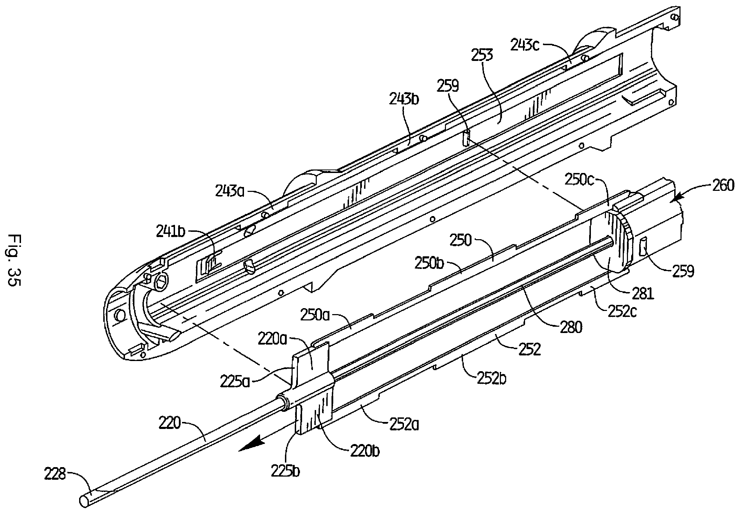

Supported within a distal end of guide housing 216 is an advancer 220. Advancer is in the form of a tube 220 which forms a cheater tube slidable within guide housing 216 and winged housing 214. Advancer tube 220, like advancer tube 39 of the embodiment of Figure 1, is in the form of a tube cut at the distal end to form a hinge 228b. The cut portion forms a casing 228. Casing 228 is somewhat cylindrical shaped with a beveled end 228a formed by the cut in the tube as shown in Figure 31A. Casing 228 forms a support housing for the covering member 104 of hole closure device 101. The casing 228 is initially mounted within the winged housing 214 in a position substantially transverse to a longitudinal axis of the winged housing 214 as shown in Figure 31A. Distal movement of the advancer tube 220 pivots the casing 228 (in the same manner as casing 28 of Figure 8A) about hinge 228b as it contacts curved surface 214c within winged housing 214 to a more linear position substantially aligned with the longitudinal axis of the winged housing 214 in the same manner as casing 28 shown in Figure 10. That is, casing 228 is pivoted from the initial transverse position of Figures 31A and 33 to the aligned position of Figures 34A and 35). This allows for pivoting movement of the elongated covering member 104 to reposition it to a more aligned position for advancement through the sheath 200. Note also that in the alignment position of casing 228, the beveled distal end 221 of advancer tube 220 abuts the beveled end 228a of casing 228 (as in casing 28 of Fig. 10).

With reference to Figure 30A, advancer tube 220 has offset wings 220a, 220b. One of the wings, e.g. wing 220a, can have indicia to indicate alignment of the casing 228. A clip 275 (see also Figure 29) provides a stop to prevent inadvertent movement of the advancer tube 220, e.g. during shipping. That is, distal edges 223a, 223b of wings 220a, 220b abut the legs 275a, 275b, respectively, of clip 275, thus preventing distal movement of advancer tube 220 unless the clip 275 is removed. Thus, clip 275 also provides a shipping lock. The upper edge 225a (as viewed in the orientation of Figure 34A) of edge 223a and a lower edge 225b of edge 223b abut internal wall 248a and 248b, respectively, of guide housing 216, to prevent further distal movement of advancer 220 as described in more detail below. That is, when advancer 220 is moved distally to the position of Figure 34A, walls 248a, 248b block further distal movement. Note the internal walls 248a, and 248b are formed by the two housing halves 240a, 240b.

A seal 258, with an opening 258a for receiving outer tube 280, prevents the ingress of fluids. The seal 258 is mounted in the distal portion of guide housing 216 within an opening in cap 257 which is fixed to housing 216 by a pair of transverse pins 256 extending into a pair of side openings 257a of cap 257.

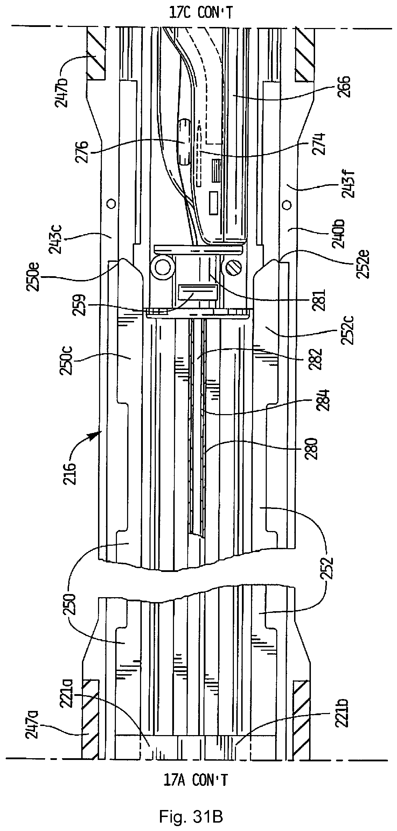

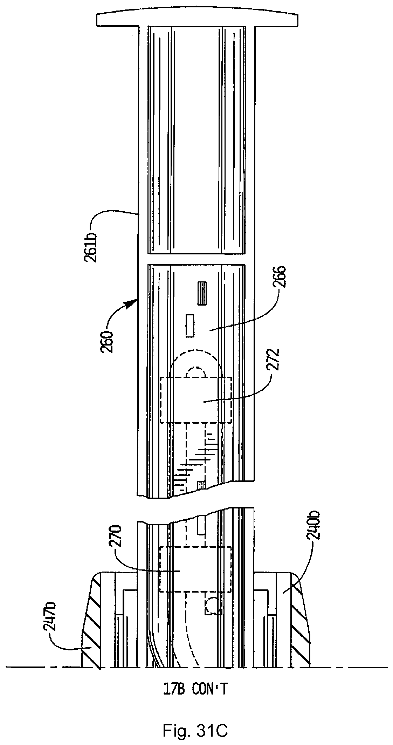

First and second rails 250, 252, extend between plunger 260 and wings 220a, 220b. More specifically, upper rail 250 (as viewed in the orientation of Figures 30A and 31A-31C) extends within guide housing 216 between the plunger 260 and wing 220a of advancer tube 220. A lower rail 252 (as viewed in the orientation of Figures 30A and 31-31C), positioned within guide housing 216 on a different plane than rail 250, extends between plunger 260 and wing 220b of advancer tube 220. As shown in Figure 33, rail 250 has a distal finger 250a, an intermediate finger 250b, and a proximal finger 250c extending upwardly to engage internal walls of the guide housing 216 discussed in detail below. Similarly, rail 252 has a distal finger 252a, an intermediate finger 252b, and a proximal finger 252c extending downwardly to engage internal walls of the guide housing 216 also discussed in detail below. The distal edges 250d, 252d of distal fingers 250a, 252a abut inwardly extending walls 243a, 243d of guide housing 216 (Figure 31A), and the proximal edges 250e, 252e, of proximal fingers 250c, 252c, abut inwardly extending walls 243c, 243f of guide housing 216 (Figure 31B) in the initial position.

The distal end of finger 250a of rail 250 is received in slot 220d of winged housing 220a; the distal end of finger 252b of rail 252 is similarly received in a slot in winged housing 220b (see Figures 33 and 37). The proximal finger 250c of rail 250 is received in slot 267a in plunger 260; proximal finger 252c of rail 252 is similarly received in a slot on the opposing side of plunger 260. Rails 250, 252 function to initially advance advancer tube 220 when plunger 260 is advanced, and then are moved out of operative engagement with wings 220a, 220b so the plunger 260 advances while the advancer tube 220 remains stationary as the outer tube 280 is advanced within advancer tube 220.

With reference to Figure 30B, plunger 260 includes housing halves 261a, 261b. Contained within the housing halves 261a, 261b are first channel housing 264, second channel housing 266 and central housing 268. These channel housings are identical to housings 40, 42, and 44 of Figures 1-26 to provide movement of the sutures 120, 122 of the hole closure device 101 and their respective engagement members to advance the retainers 110, 112 of the hole closure device 101 and therefore for brevity are not further described herein or further labeled in the drawings. Knife 274 is supported in the slots in the channel housings 264, 266 and 268. Knife 274 is identical in structure and function to knife 54 of Figure 2A. A shroud 276 is positioned above the knife 274 (as viewed in the orientation of Figure 30B) to prevent inadvertent contact of the sutures 120, 122 with the knife 274 which can cause premature severing of the sutures 120, 122 of the hole closure device 101. (A shroud can also be provided above knife 54 in the embodiment of Figure 1). Grommets 270, 272, having engaging portions to form blocking or abutment members for the engagement members 125, 123 on the end of the sutures 120, 122 of the hole closure device 101, are identical to grommets 49, 48, respectively, of Figure 2A and therefore for brevity are not discussed in detail below since the description of the function of grommets 48, 49, and the passage of engagement members 125, 123 are fully applicable to this embodiment of Figure 30B. Consequently, when device 212 is retracted after delivery of the hole closure device 101 to the vessel, the sutures and engagement members pass through the channels in housing 264, 266 and 268 in the same manner as in the embodiment of Figures 1-26 to advance the retainers toward the covering member to secure the hole closure device and to sever the sutures.

With reference to Figure 30A, end cap 281 of inner tube 282 is secured within opening 260a of plunger 260 by a snap or friction fit. Inner tube 282 is received in the lumen of outer tube 280. A reinforcing tube 284, preferably composed of stiffer material, is concentrically interposed between the inner tube 282 and outer tube 280. Outer tube 280 is attached to the end cap 281 of plunger 260 so that distal movement of plunger 260 advances the outer tube 280 through the lumen in advancer tube 220 to contact spherical retainer 110 of the closure device 101 to advance the closure device 101 through the sheath 200 and into the vessel. The inner tube 282 has a pair of lumens to receive respective sutures 120, 122.

Proximal and distal sleeves 247b, 247a surround the housing halves 240a, 240b of the guide housing 216 to secure them together.

In use, in the initial position of Figures 31a-31c (and Figure 33), plunger 260 is in its proximalmost position and advancer tube 220 is in the proximalmost position as edges 223a, 223b of wings 220a, 220b, respectively, abut clip 275. Thus, in this initial position, with the locking clip 275 in place, distal movement of plunger 260 is blocked. Note in this position, casing 228 (and hole covering member 104 contained therein) are in the transverse position. Rails 250, 252 which extend between plunger 260 and wings 220a, 220b, are positioned such that edge 250e of proximal finger 250c of rail 250 is in contact with the edge of proximal radially inwardly extending internal wall 243c of housing 216 and edge 252e of proximal finger 252c of rail 252 is in contact with the edge of proximal radially inwardly extending wall 243f of housing 216, best shown in Figure 31B. Additionally, edge 250d of distal finger 250a is in contact with the edge of distal radially inwardly extending internal wall 243a of housing 216 and edge 252d of distal finger 252a is in contact with the edge of distal radially inwardly extending wall 243d of housing 216. Thus, the fingers 250a, 250b, 250c of rail 250 are spaced from the spaces between walls 243a, 243b and 243c, and the fingers 252a, 252b and 252c of rail 252 are spaced from the spaces between walls 243d, 243e and 243f.

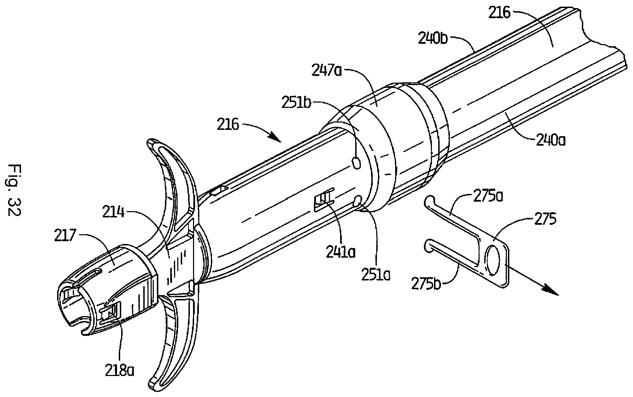

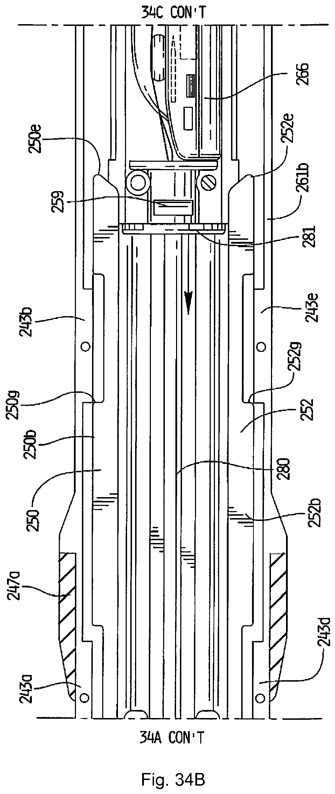

To actuate the delivery instrument 212 to advance the plunger 260 to move the advancer tube 220 distally in the first stage of operation, clip 275 is manually removed from the openings 251a, 251b of guide housing 216 (Figure 32) and plunger 260 is advanced distally. Such distal advancement advances the advancer tube 220 due to the engagement of rails 250, 252 with wings 220a, 220b of advancer tube 220. Advancement of the advancer tube 220 causes casing 228 to pivot from its transverse position to the longitudinally aligned position of Figures 34A and 35 as the casing 228 is forced against internal curved wall 214c. The plunger 260 is advanced so that aligned casing 228 is moved through winged housing 214 and into a proximal region of sheath 200. Note that the longitudinal alignment of casing 228 pivots covering member 104 of hole closure device 101 to a more aligned position for delivery through the sheath 200 to the vessel lumen. Note that roller pins 259 (Figures 34B and 35) on opposing sides of plunger 260 ride within longitudinal track 253 of housing 240b and a similar longitudinal track in housing 240a, thereby acting as low friction bearings within the tracks in housing halves 240a, 240b for smoother motion of the plunger 260 within guide housing 216. This advanced position of tube 220 and intermediate position of plunger 260 are shown in Figures 34A-34C and 35.

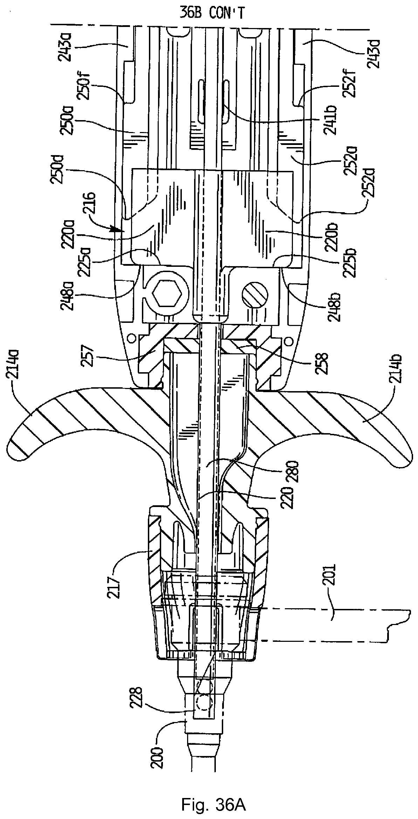

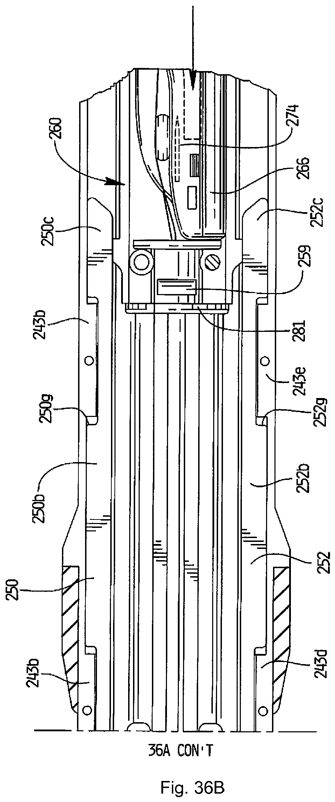

Note that plunger 260 advances advancer tube 220 until the distal edges 225a, 225b of wings 220a, 220b abut edges 248a, 248b of housing 216 as shown in Figures 34A. In this position, plunger 260 has advanced to an intermediate region of guide housing 216. As the advancer tube 220 is thus blocked against further distal movement by edges 248a, 248b, further distal movement of plunger 260 does not advance advancer tube 220. Instead, as plunger 260 is advanced, distal edges 250d and 252d of rails 250, 252, respectively, and 250g of rail 250 have moved past the distal edges of respective distal radially extending walls 243a and 243d and proximal edges 250e, 252e of rails 250, 252 respectively, have moved past the distal edges of the respectively radially extending proximal walls 243c, 243f. However, proximal edge 250f of distal finger 250a and proximal edge 252f of distal finger 252 remain engaged with walls 243a, 243d. Further, proximal edges 250g, 252g of intermediate fingers 250b, 252b, respectively, are engaged with distal edges of walls 243b, 243e (Figure 34B). Upon further distal movement of plunger 260, proximal, intermediate and distal fingers 250a, 250b and 250c of rail 250 extend into the respective spaces between walls 243a, 243b and 243c and the proximal, intermediate, and distal fingers 252a, 252b and 252c of rail 252 extend into the respective spaces between the walls 243d, 243e and 243f as shown in Figures 36A, 36B and 37 as edges 250f, 252f and 250g, 252g clear the respective walls. When this occurs, rail 250 comes out of the slot 220d in wings 220a and rail 252 comes out of the slot in wing 220b so the rails 250, 252 are disengaged from wings 220a, 220b and thus the advancer 220 is no longer in operative engagement with plunger 260. Note rails 250, 252 ride within ramps in the wings 220, 220b. That is, in this position of rails 250, 252, advancement of plunger 260 no longer advances advancer 220 and instead, advancement of plunger 260 moves the outer tube 280 with respect to the advancer 220 which is now stationary.

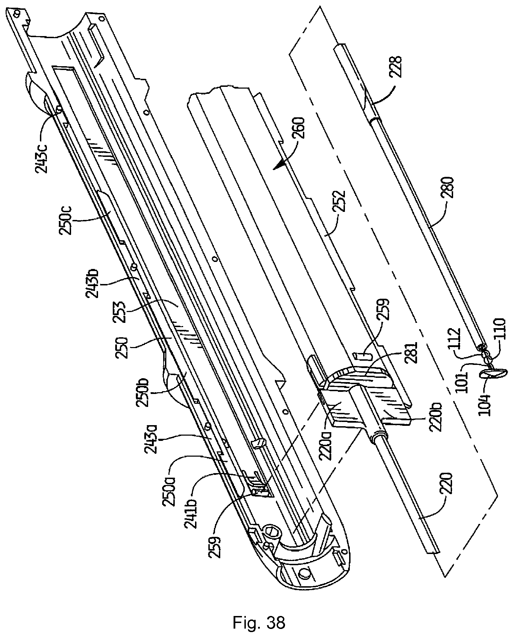

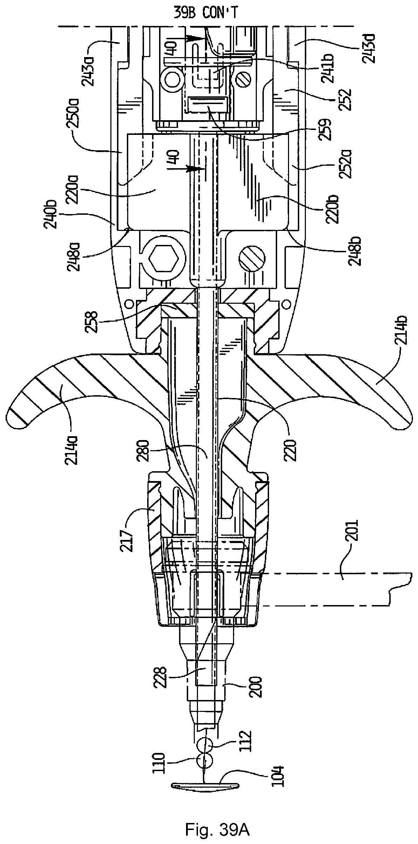

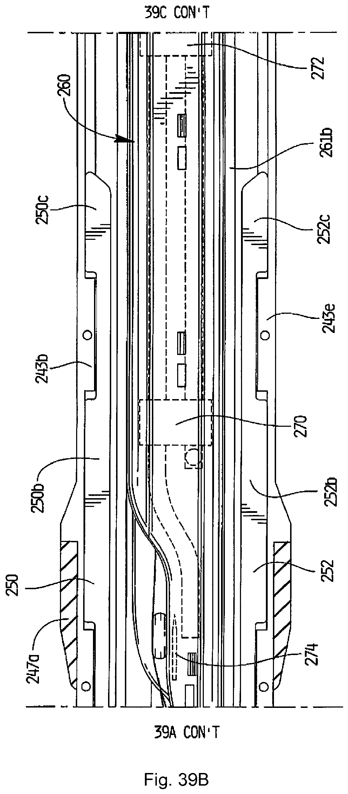

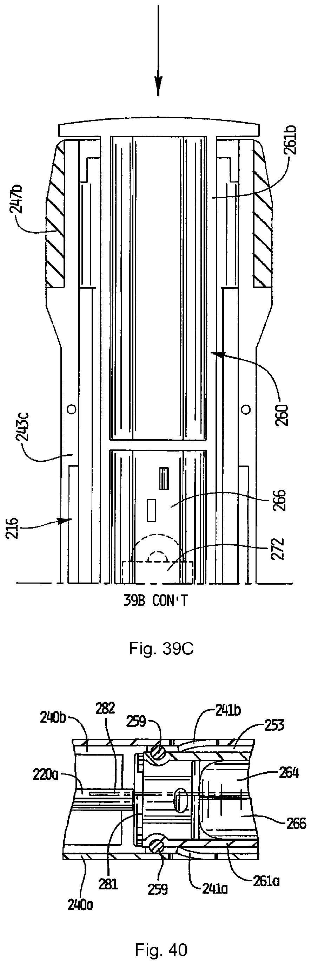

Upon further distal advancement of plunger 260 to its distalmost position as shown in Figures 39A-39C and 38, outer tube 280 moves within stationary advancer tube 220 as the wings 220a, 220b are blocked by walls 248a, 248b from further advancement, and the rails 250, 252 are no longer in operative engagement with the wings 220a, 220b. As outer tube 280 is advanced through winged housing 214 and into the sheath 200, it continues to advance the hole closure device 101 through the sheath 200 and into the vessel as it engages retainer 110. That is, full distal advancement of plunger 260 advances the hole closure device 101 from the distal end of the sheath 200. This position is shown in Figure 35. In this distalmost position, pins 259 are locked by locking tabs 241a, 241b to prevent proximal movement thereof. That is, when plunger 260 has completed its stroke, pins 259 have bypassed locking tabs 241a, 241b and are now blocked from proximal movement by these tabs 241a, 241b.

Once the plunger 260 is fully advanced and locked in its distal position, i.e., blocked from proximal movement, the delivery instrument 212 is retracted in the same manner as delivery instrument 100 of Figures 1-26 to advance the retainers 110, 112 toward covering member 104 and to automatically sever the sutures 120, 122 by knife 274 as the sutures 120, 122 and engagements members slide within the channels in the housings of Figure 30B.

## Claims

1. A surgical delivery instrument for delivering into a vessel a vascular hole closure device (101) having a hole covering member (104), the delivery instrument (10; 212) comprising a housing (22; 214), an advancer (39; 220) movable within the housing, and a plunger (12; 260), the advancer (39; 220) having a first portion and a distal portion (26; 228) hingedly connected to the first portion and forming a casing for supporting a hole covering member the plunger being movable subsequent to the movement of the advancer (39, 220) to advance said hole covering member from the housing, **characterised in that** the housing includes an angled inner surface (22c; 214c) engageable by the casing for pivoting the same within the housing (22; 214), distal movement of the advancer pivoting the casing within the housing from an angled position relative to the first portion to a more aligned position relative to the first portion.

2. A surgical delivery instrument as claimed in claim 1, wherein the plunger (12; 260) includes a tube (14, 280) and a handle portion (13) positioned proximally of the tube, and movement of the handle portion distally advances the tube within a lumen of the advancer (39; 220).

3. A surgical delivery instrument as claimed in claim 1 or 2, further comprising a distal stop (31) to limit distal movement of the advancer (39).

4. A surgical delivery instrument as claimed in any preceding claim, further comprising a first actuator (37) for moving the advancer (39; 220) distally, the first actuator movable independently of the plunger (12; 260).

5. A surgical delivery instrument as claimed in any preceding claim, wherein the plunger (260) is configured such that initial advancement thereof moves the advancer (220) distally and subsequent advancement thereof advances the covering member (104) from the housing.

6. A surgical delivery instrument as claimed in any preceding claim, further comprising first and second rails (250, 252) operatively connecting the advancer (220) and the plunger (260), wherein the first and second rails are operatively disassociated from the plunger when the advancer is moved to a distal position into contact with a stop (248a, 248b).

7. A kit comprising a surgical delivery instrument as claimed in any preceding claim and a vascular hole closure device (101) including a hole covering member (104), wherein the hole closure device (101) further includes first and second flexible members (120, 122), the first flexible member (120) having a first engagement member (125) and the second flexible member (122) having a second engagement member (123), wherein the plunger (12; 260) has first and second longitudinally extending openings (40a, 58a) and first and second engaging portions (48a, 49a), the first engaging portion (48a) limiting movement of the first engagement member (125) and the second engaging portion (49a) limiting movement of the second engagement member (123), wherein the first engagement member is held by the first engaging portion until a predetermined force is applied to the first engagement member during placement of the closure device (101) at a target site and the second engagement member is held by the second engaging portion until a predetermined force is applied to the second engagement member during placement of the closure device at the target site.

8. A kit as claimed in claim 7, wherein the first engaging portion comprises a first opening (48a) in a first grommet (48; 270) aligned with the first longitudinally extending opening (40a) and the second engaging portion comprises a second opening (49a) in a second grommet (49; 272) aligned with the second longitudinally extending opening (58a).

9. A kit as claimed in claim 8, wherein the first grommet (48; 270) has a third opening (48b) and the second grommet (49; 272) has a fourth opening (49b), wherein the first engagement member (125) is passable through the first opening (48a) when a first force is applied and subsequently passable through the third opening (48b) when a subsequent force is applied and the second engagement member (123) is passable through the second opening (49a) when a second force is applied and subsequently passable through the fourth opening (49b) when a subsequent force is applied.

10. A kit as claimed in claim 7, 8 or 9, wherein the first flexible member (120) is a suture and the second flexible member (122) is a suture, and the delivery instrument (10; 212) further comprises a cutting member (54; 274) positioned within the housing (22; 214) for automatically severing the sutures.

11. A kit as claimed in any one of claims 7 to 10, wherein the covering member (104) of the vascular hole closure device (101) is at a distal end for positioning internal of a vessel and the device further includes a first and a second retainer (110, 112) for positioning external of the vessel, the first flexible member (120) extending between the covering member and the first retainer and the first engagement member positioned at a proximal portion of the first flexible member, and the second flexible member (122) extending between the covering member and the second retainer and the second engagement member positioned at a proximal portion of the second flexible member, wherein proximal movement of the delivery instrument (10; 212) advances the first retainer and the second retainer toward the covering member.

## Patentansprüche

1. Chirurgisches Abgabeinstrument für die Abgabe einer Verschließvorrichtung für ein Gefäßloch (101) in ein Gefäß, die ein Lochabdeckungselement (104) aufweist, wobei das Abgabeinstrument (10; 212) ein Gehäuse (22; 214), eine Vorschubeinheit (39; 220), die in dem Gehäuse beweglich ist, und einen Kolben (12; 260) umfasst, wobei die Vorschubeinheit (39; 220) einen ersten Abschnitt und einen distalen Abschnitt (26; 228) aufweist, der mit dem ersten Abschnitt klappbar verbunden ist und eine Ummantelung für die Unterstützung eines Lochabdeckungselements bildet, der Kolben im Anschluss an die Bewegung der Vorschubeinheit (39, 220) beweglich ist, um das Lochabdeckungselement aus dem Gehäuse vorzuschieben,
**dadurch gekennzeichnet, dass** das Gehäuse eine abgewinkelte innere Oberfläche (22c; 214c) einschließt, die durch die Ummantelung für die Drehung derselben innerhalb des Gehäuses (22; 214) eingreifen kann, eine distale Bewegung der Vorschubeinheit die Ummantelung innerhalb des Gehäuses von einer abgewinkelten Position relativ zum ersten Abschnitt zu einer angepassteren Position relativ zum ersten Abschnitt dreht.

2. Chirurgisches Abgabeinstrument nach Anspruch 1, wobei der Kolben (12; 260) ein Rohr (14, 280) und einen Griffabschnitt (13), der proximal zum Rohr positioniert ist, einschließt und eine Bewegung des Griffabschnitts das Rohr innerhalb eines Hohlraums der Vorschubeinheit (39; 220) distal vorschiebt.

3. Chirurgisches Abgabeinstrument nach Anspruch 1 oder 2, das weiter einen distalen Stopper (31) umfasst, um eine distale Bewegung der Vorschubeinheit (39) zu begrenzen.

4. Chirurgisches Abgabeinstrument nach einem vorstehenden Anspruch, das weiter einen ersten Aktuator (37) für die distale Bewegung der Vorschubeinheit (39; 220) umfasst, wobei der erste Aktuator unabhängig vom Kolben (12; 260) beweglich ist.

5. Chirurgisches Abgabeinstrument nach einem vorstehenden Anspruch, wobei der Kolben (260) derart konfiguriert ist, dass anfänglicher Vorschub davon die Vorschubeinheit (220) distal bewegt und anschließender Vorschub davon das Abdeckungselement (104) aus dem Gehäuse vorschiebt.

6. Chirurgisches Abgabeinstrument nach einem vorstehenden Anspruch, das weiter eine erste und zweite Schiene (250, 252) umfasst, die die Vorschubeinheit (220) und den Kolben (260) funktionsfähig verbinden, wobei die erste und zweite Schiene von dem Kolben funktionsfähig getrennt werden, wenn die Vorschubeinheit zu einer distalen Position im Kontakt mit einem Stopper (248a, 248b) bewegt wird.

7. Kit, der ein chirurgisches Abgabeinstrument nach einem vorstehenden Anspruch und eine Verschließvorrichtung für ein Gefäßloch (101), die ein Lochabdeckungselement (104) einschließt, umfasst, wobei die Verschließvorrichtung für ein Gefäßloch (101) weiter ein erstes und zweites flexibles Element (120, 122) einschließt, wobei das erste flexible Element (120) ein erstes Eingreifelement (125) aufweist und das zweite flexible Element (122) ein zweites Eingreifelement (123) aufweist, wobei der Kolben (12; 260) eine erste und zweite, sich länglich erstreckende Öffnung (40a, 58a) und einen ersten und zweiten eingreifenden Abschnitt (48a, 49a) aufweist, der erste eingreifende Abschnitt (48a) eine Bewegung des ersten Eingreifelements (125) begrenzt und der zweite eingreifende Abschnitt (49a) eine Bewegung des zweiten Eingreifelements (123) begrenzt, wobei das erste Eingreifelement von dem ersten eingreifenden Abschnitt gehalten wird, bis eine vorgegebene Kraft auf das erste Eingreifelement während der Positionierung der Verschließvorrichtung (101) an einer Zielstelle ausgeübt wird, und das zweite Eingreifelement von dem zweiten eingreifenden Abschnitt gehalten wird, bis eine vorgegebene Kraft auf das zweite Eingreifelement während der Positionierung der Verschließvorrichtung (101) an der Zielstelle ausgeübt wird.

8. Kit nach Anspruch 7, wobei der erste eingreifende Abschnitt eine erste Öffnung (48a) in einer ersten Durchführung (48; 270) umfasst, die mit der ersten, sich länglich erstreckenden Öffnung (40a) ausgerichtet ist, und der zweite eingreifende Abschnitt eine zweite Öffnung (49a) in einer zweiten Durchführung (49; 272) umfasst, die mit der zweiten, sich länglich erstreckenden Öffnung (58a) ausgerichtet ist.

9. Kit nach Anspruch 8, wobei die erste Durchführung (48; 270) eine dritte Öffnung (48b) aufweist und die zweite Durchführung (49; 272) eine vierte Öffnung (49b) aufweist, wobei das erste Eingreifelement (125) durch die erste Öffnung (48a) durchführbar ist, wenn eine erste Kraft ausgeübt wird, und anschließend durch die dritte Öffnung (48b) durchführbar ist, wenn eine anschließende Kraft ausgeübt wird, und das zweite Eingreifelement (123) durch die zweite Öffnung (49a) durchführbar ist, wenn eine zweite Kraft ausgeübt wird, und anschließend durch die vierte Öffnung (49b) durchführbar ist, wenn eine anschließende Kraft ausgeübt wird.

10. Kit nach Anspruch 7, 8 oder 9, wobei das erste flexible Element (120) ein Nahtmaterial ist und das zweite flexible Element (122) ein Nahtmaterial ist und das Abgabeinstrument (10; 212) weiter ein Schneidelement (54; 274) umfasst, das innerhalb des Gehäuses (22; 214) für die automatische Durchtrennung der Nahtmaterialien positioniert ist.

11. Kit nach einem der Ansprüche 7 bis 10, wobei das Abdeckungselement (104) der Verschließvorrichtung für ein Gefäßloch (101) an einem distalen Ende für die Positionierung innerhalb eines Gefäßes vorliegt und die Vorrichtung weiter ein erstes und ein zweites Halteelement (110, 112) für die Positionierung außerhalb des Gefäßes einschließt, sich das erste flexible Element (120) zwischen dem Abdeckungselement und dem ersten Halteelement und dem ersten Eingreifelement, das an einem proximalen Abschnitt des ersten flexiblen Elements positioniert ist, erstreckt und sich das zweite flexible Element (122) zwischen dem Abdeckungselement und dem zweiten Halteelement und dem zweiten Eingreifelement, das an einem proximalen Abschnitt des zweiten flexiblen Elements positioniert ist, erstreckt, wobei eine proximale Bewegung des Abgabeinstruments (10; 212) das erste Halteelement und das zweite Halteelement in Richtung des Abdeckungselements vorschiebt.

## Revendications

1. Instrument d'introduction chirurgical pour introduire dans un vaisseau un dispositif d'obturation de trou vasculaire (101) comportant un élément de recouvrement de trou (104), l'instrument d'introduction (10 ; 212) comprenant un logement (22 ; 214), un dispositif d'avancée (39 ; 220) déplaçable à l'intérieur du logement, et un piston (12 ; 260), le dispositif d'avancée (39 ; 220) comportant une première partie et une partie distale (26 ; 228) raccordée de manière articulée à la première partie et formant un boîtier pour supporter un élément de recouvrement de trou, le piston étant déplaçable suite au déplacement du dispositif d'avancée (39, 220) pour faire avancer ledit élément de recouvrement de trou à partir du logement,
**caractérisé en ce que** le logement comporte une surface interne inclinée (22c ; 214c) avec laquelle le boîtier peut s'engager pour faire pivoter celui-ci à l'intérieur du logement (22 ; 214), un mouvement distal du dispositif d'avancée faisant pivoter le boîtier à l'intérieur du logement d'une position inclinée par rapport à la première partie à une position plus alignée par rapport à la première partie.

2. Instrument d'introduction chirurgical selon la revendication 1, dans lequel le piston (12 ; 260) comporte un tube (14, 280) et une partie de poignée (13) positionnée proximalement au tube, et le déplacement de la partie de poignée fait avancer distalement le tube à l'intérieur d'une lumière du dispositif d'avancée (39 ; 220).

3. Instrument d'introduction chirurgical selon la revendication 1 ou 2, comprenant en outre une butée distale (31) pour limiter le déplacement distal du dispositif d'avancée (39).

4. Instrument d'introduction chirurgical selon l'une quelconque des revendications précédentes, comprenant en outre un premier actionneur (37) pour déplacer distalement le dispositif d'avancée (39 ; 220), le premier actionneur étant déplaçable indépendamment du piston (12 ; 260).

5. Instrument d'introduction chirurgical selon l'une quelconque des revendications précédentes, dans lequel le piston (260) est configuré de telle sorte qu'une avancée initiale de celui-ci déplace le dispositif d'avancée (220) distalement et qu'une avancée ultérieure de celui-ci fasse avancer l'élément de recouvrement (104) à partir du logement.

6. Instrument d'introduction chirurgical selon l'une quelconque des revendications précédentes, comprenant en outre des premier et second rails (250, 252) raccordant opérationnellement le dispositif d'avancée (220) et le piston (260), dans lequel les premier et second rails sont séparés opérationnellement du piston quand le dispositif d'avancée est déplacé jusqu'à une position distale en contact avec une butée (248a, 248b).

7. Kit comprenant un instrument d'introduction chirurgical selon l'une quelconque des revendications précédentes et un dispositif d'obturation de trou vasculaire (101) comportant un élément de recouvrement de trou (104), dans lequel le dispositif d'obturation de trou (101) comporte en outre des premier et second éléments souples (120, 122), le premier élément souple (120) comportant un premier élément d'engagement (125) et le second élément souple (122) comportant un second élément d'engagement (123), dans lequel le piston (12 ; 260) comporte des première et seconde ouvertures s'étendant longitudinalement (40a, 58a) et des première et seconde parties d'engagement (48a, 49a), la première partie d'engagement (48a) limitant le déplacement du premier élément d'engagement (125) et la seconde partie d'engagement (49a) limitant le déplacement du second élément d'engagement (123), dans lequel le premier élément d'engagement est retenu par la première partie d'engagement jusqu'à ce qu'une force prédéterminée soit appliquée au premier élément d'engagement durant le placement du dispositif d'obturation (101) à un site cible et le second élément d'engagement est retenu par la seconde partie d'engagement jusqu'à ce qu'une force prédéterminée soit appliquée au second élément d'engagement durant le placement du dispositif d'obturation au site cible.

8. Kit selon la revendication 7, dans lequel la première partie d'engagement comprend une première ouverture (48a) dans un premier passe-fil (48 ; 270) alignée avec la première ouverture s'étendant longitudinalement (40a) et la seconde partie d'engagement comprend une deuxième ouverture (49a) dans un second passe-fil (49 ; 272) alignée avec la seconde ouverture s'étendant longitudinalement (58a).

9. Kit selon la revendication 8, dans lequel le premier passe-fil (48 ; 270) a une troisième ouverture (48b) et le second passe-fil (49 ; 272) a une quatrième ouverture (49b), dans lequel le premier élément d'engagement (125) peut passer à travers la première ouverture (48a) quand une première force est appliquée puis passer par la troisième ouverture (48b) quand une force ultérieure est appliquée et le second élément d'engagement (123) peut passer à travers la deuxième ouverture (49a) quand une seconde force est appliquée puis passer par la quatrième ouverture (49b) quand une force ultérieure est appliquée.

10. Kit selon la revendication 7, 8 ou 9, dans lequel le premier élément souple (120) est une suture et le second élément souple (122) est une suture, et l'instrument d'introduction (10 ; 212) comprend en outre un élément de coupe (54 ; 274) positionné à l'intérieur du logement (22 ; 214) pour trancher automatiquement les sutures.

11. Kit selon l'une quelconque des revendications 7 à 10, dans lequel l'élément de recouvrement (104) du dispositif d'obturation de trou vasculaire (101) se trouve à l'extrémité distale pour le positionnement à l'intérieur d'un vaisseau et le dispositif comporte en outre un premier et un second dispositif de retenue (110, 112) pour le positionnement à l'extérieur du vaisseau, le premier élément souple (120) s'étendant entre l'élément de recouvrement et le premier dispositif de retenue et le premier élément d'engagement étant positionné au niveau d'une partie proximale du premier élément souple, et le second élément souple (122) s'étendant entre l'élément de recouvrement et le second dispositif de retenue et le second élément d'engagement positionné au niveau d'une partie proximale du second élément souple, dans lequel le déplacement proximal de l'instrument d'introduction (10 ; 212) fait avancer le premier dispositif de retenue et le second dispositif de retenue vers l'élément de recouvrement.
